# EUROPEAN PATENT APPLICATION

(11) **EP 4 134 674 A1**
(43) Date of publication of application: **15.02.2023**
(21) Application number: 22189863.8
(22) Date of filing: 11.08.2022
(51) Int. Cl.: G01N 33/68, C07K 14/415

(54) **METHODS AND REAGENTS FOR DETECTING AN ANTIBODY TO A SES IPR-10 ALLERGEN**

(30) Priority: 13.08.2021 EP 21191320
(71) Applicant: Euroimmun Medizinische Labordiagnostika AG, 23560 Lübeck (DE)
(72) Inventor: Brix, Bettina, 23560 Lübeck (DE); Suer, Waltraud, 23560 Lübeck (DE); Morgenroth, Friedrich, 23560 Lübeck (DE); Rohwer, Stefanie, 23560 Lübeck (DE); Rossnagel, Madlen, 23560 Lübeck (DE)

(57) **Abstract**

The present invention relates to a polypeptide comprising a Ses iPR-10 allergen from sesame characterized by SEQ ID NO1 or a variant thereof, wherein the polypeptide is isolated, purified, diluted or immobilized, a diagnostically useful carrier comprising the polypeptide, a kit comprising the carrier, wherein the kit further comprises one or more from the group comprising a suitable water-tight vessel for contacting the diagnostically useful carrier with the sample, a negative control, positive control, a calibrator, a washing buffer, a dilution buffer, a means for detecting an IgG and/or IgE class antibody to a Ses iPR-10 allergen from sesame characterized by SEQ ID NO1, a means for capturing class IgG and/or IgE class antibodies, a method for diagnosing or aiding in the diagnosis of an allergy comprising the step detecting in a sample from a subject the presence or absence of an antibody binding specifically to a Ses iPR-10 allergen from sesame characterized by SEQ ID NO1, and a use of the polypeptide for detecting an antibody binding specifically to a Ses iPR-10 allergen from sesame characterized by SEQ ID NO1.

## Description

The present invention relates to a polypeptide comprising a Ses iPR-10 allergen from sesame characterized by SEQ ID NO1 a variant thereof, wherein the polypeptide is isolated, purified or immobilized, a diagnostically useful carrier comprising the polypeptide, a kit comprising the carrier, wherein the kit further comprises one or more from the group comprising a suitable water-tight vessel for contacting the diagnostically useful carrier with the sample, a negative control, positive control, a calibrator, a washing buffer, a dilution buffer, a means for detecting an IgG and/or IgE class antibody to a Ses iPR-10 allergen from sesame characterized by SEQ ID NO1, a means for capturing class IgG and/or IgE class antibodies, a method for diagnosing or aiding in the diagnosis of an allergy comprising the step detecting in a sample from a subject the presence or absence of an antibody binding specifically to a Ses iPR-10 allergen from sesame characterized by SEQ ID NO1, and a use of the polypeptide for detecting an antibody binding specifically to a Ses iPR-10 allergen from sesame characterized by SEQ ID NO1.

Allergic reactions to plants range from mild to systemic reactions, including severe anaphylactic reactions. Apart from allergic reactions upon consumption of recognizable plant products, allergens can be unrecognizable components in a variety of foodstuffs where their nature and hence the danger of allergic reactions is hidden.

In spite of the high prevalence of plant food allergy, few options are available for treatment, and avoidance is often the only possible option. Of course, this requires precise information regarding the nature of the antigen(s) to which the patient reacts.

A range of tests are commercially available, in particular regarding the most prevalent plant food components, in particular peanut, hazelnut and walnut. For example, Ara h 7 isotype 7.0201 has been identified as a major allergen in peanuts (US20180231567A1). An assay based on a novel macadamia allergen is described in US10842865.

Such diagnostic tests for plant food allergies involve the detection of IgE and/or IgG antibodies from patients with a specificity with regard to proteins from an allergen source. However, a positive IgE or IgG test, for example IgE sensitization, does not always correlate with clinical manifestations. In other words, if IgE or IgG class antibodies to one allergen are detected, this observation is not sufficient to provide a conclusive diagnosis.

However, it is a first indication suggesting that the allergen source tested is more likely to be relevant than others. In clinical practice, a medical doctor's diagnosis of an allergy is usually based on a positive IgE or IgG sensitization for the relevant allergen source and a convincing clinical history of allergic reactions to this allergen. In addition, the results of complementary tests such as a skin prick test (SPT), performed by topical application of the specific extract on the skin of the patient, an oral food challenge and/or a basophile activation test may be carried out.

Following successful identification of an allergy to a specific allergen, a treatment may follow. While anti-histamines may be used to ameliorate treatment, a long-term and curative treatment can be performed with specific immunotherapy based on the controlled administration of the allergen. Although the exact mechanisms are not fully known, such a specific activation of the immune system alleviates the symptoms upon subsequent environmental exposure to the same allergen. Successful treatment has been demonstrated for various plant pollen and animal allergens. However, in some cases avoiding an allergen which has been identified as the causative agent of the allergy is the only feasible option.

Sesame allergy is an increasingly common plant food allergy. It is now estimated that at least 0.2% of children and adults are allergic to sesame, which approaches the prevalence rates of a number of well-known allergens such as soy and pistachio. It can cause severe allergic reactions with multiple organ system involvement (also known as anaphylaxis). As sesame is not listed as a major food allergen, consumers may be vulnerable to accidental exposure to this allergen. Sesame is present in a wide, and growing, variety of food products in the form of seeds, oils and pastes (i.e. tahini). Some cosmetics, medications, supplements and pet food also contain sesame.

A sesame allergy can be diagnosed by detecting specific IgE class antibodies based on sesame extracts or 2S albumin, Ses i 1, the major allergen. However, this leads to false negative results in around 30% of sesame allergic patients (Ehlers, A. M., Rossnagel, M., Brix, B., Blankestijn, M. A., Le, T. M., Suer, W., Otten, H. G., & Knulst, A. C. (2019). Sesame oleosins are minor allergens. Clinical and translational allergy, 9, 32. https://doi.org/10.1186/s13601-019-0271-x). Moreover, plant extracts are not the preferred source of antigens for tests, because they are hard to standardize, meaning that extracts prepared according to different protocols or based on different batches of raw materials yield different results. Moreover, minor antigens may be masked by other components or their concentration may be insufficient.

Therefore, the use of purified recombinant sesame allergens is preferred. However, there is a shortage of diagnostically useful sesame allergens. Oleosins, a class of major allergens in peanuts and hazelnuts, were shown to be only minor allergens in sesame allergy without diagnostic value (Ehlers, A.M., Rossnagel, M., Brix, B. et al. Sesame oleosins are minor allergens. Clin Transl Allergy 9, 32 (2019). https://doi.org/10.1186/s13601-019-0271-x). There is a considerable need for additional diagnostic tools to minimize the need for food challenges.

Therefore, the problem underlying the present invention is to provide reagents and methods for the diagnosis or for aiding in the diagnosis of an allergy, preferably a plant food allergy, more preferably a sesame allergy, or a plant PR-10 allergy.

Another problem underlying the present invention is to provide reagents and methods for the detection of sesame-specific antibodies, preferably IgG and IgE antibodies, preferably diagnosing or aiding in the diagnosis of a plant food allergy, more preferably a sesame allergy, or a plant PR-10 allergy.

Another problem is to provide methods and reagents for the diagnosis or aiding in the diagnosis of sesame allergy or a plant PR-10 allergy having an improved diagnostic reliability, in particular sensitivity and/or specificity, compared to state of the art methods and reagents. Preferably, the diagnostic gap of 30% misdiagnosed patients with false negative samples is to be considered. The problem may involve increasing the sensitivity of the detection compared to state of the art assays such as those based on sesame extract and/or Ses i 1 allergen.

Another problem underlying the present invention is to provide reagents and methods for the differential diagnosis or aiding in the differential diagnosis of sesame allergy, whereby a sesame allergy can be distinguished from other plant food allergies, in particular an allergy against nuts or seeds.

Another problem underlying the present invention is to identify or aid in identifying plant materials which should be avoided by a patient suffering from an allergy, preferably a plant food allergy, more preferably sesame allergy or a plant PR-10 allergy.

The problem underlying the present invention is solved by the subject-matter of the attached independent and dependent claims

In a first aspect, the problem underlying the present invention is solved by a polypeptide comprising a Ses iPR-10 allergen from sesame characterized by SEQ ID NO1 or a variant thereof, wherein the polypeptide is isolated, purified or immobilized.

In a second aspect, the problem is solved by a diagnostically useful carrier comprising the polypeptide according to the present invention.

In a preferred embodiment, the carrier is selected from the group comprising a bead, a test strip, a microtiter plate, a plastic surface, preferably polystyrene surface, a branched cellulose-based polymer, a microarray, a blot, preferably from the group comprising western blot, line blot and dot blot, a glass surface, a slide, a biochip and a membrane, and is most preferably a microtiter plate or a line blot.

In a preferred embodiment, the polypeptide is in complex with an antibody from the sample of a patient, preferably an IgG, more preferably IgG4, or an IgE class antibody.

In a third aspect, the problem underlying the present invention is solved by a kit comprising the carrier according to the present invention, wherein the kit further comprises one or more, preferably all from the group comprising a suitable water-tight vessel for contacting the diagnostically useful carrier with the sample, a negative control, positive control, a calibrator, preferably a set of calibrators, a washing buffer, a dilution buffer, a means for detecting an antibody to a Ses iPR-10 allergen from sesame characterized by SEQ ID NO1, preferably an IgG and/or IgE class antibody, wherein the antibody is preferably a mammalian, more preferably a human antibody, an antibody to a Ses iPR-10 allergen from sesame characterized by SEQ ID NO1, a means for capturing the antibody, preferably an IgG and/or IgE class antibody,, more preferably human, and a polypeptide comprising a Ses iPR-10 allergen from sesame characterized by SEQ ID NO1, preferably with a detectable label.

In a preferred embodiment, the carrier is a test stripe comprising spatially separate reagents which include a Ses iPR-10 allergen from sesame characterized by SEQ ID NO1 or a variant thereof, and one or more from the group comprising one or more calibrators, an anti-CCD-IgE control, a reagent indicating the presence of a secondary antibody, preferably a secondary antibody recognizing IgG, more preferably IgG4, or IgE class antibodies, an indicator band and one or more calibration or cut off bands.

In a 4^{th} aspect, the problem is solved by a pharmaceutical composition comprising a Ses iPR-10 allergen from sesame characterized by SEQ ID NO1 or a variant thereof, preferably further comprising an adjuvant.

In a 5^{th} aspect, the problem is solved by a method for diagnosing or aiding in the diagnosis of an allergy comprising the step detecting in a sample from a subject the presence or absence of an antibody binding specifically to a Ses iPR-10 allergen from sesame characterized by SEQ ID NO1.

In a preferred embodiment, the sample is selected from the group comprising whole blood, saliva, serum and plasma.

In a 6^{th} embodiment, the method comprising the step contacting a medical or diagnostic device comprising a polypeptide comprising a Ses iPR-10 allergen from sesame characterized by SEQ ID NO1 or a variant thereof with a buffered solution comprising an antibody binding specifically to a Ses iPR-10 allergen from sesame characterized by SEQ ID NO1, which solution is not a sample from a patient in need of a diagnosis comprising an unknown concentration of said antibody, wherein preferably
a) the concentration of the antibody in the solution is known, and/or
b) the antibody is a recombinant antibody and/or
c) the medical or diagnostic device is contacted with two or more solutions comprising the antibody, wherein the two or more solutions have a different concentration of the antibody, and/or
d) the antibody is recognized by secondary antibodies binding specifically to IgE class antibodies,
followed by detection of a signal which indicates whether or not the antibody has bound to the polypeptide, optionally a signal relating to the concentration of the antibody in the solution or solutions.

In a 7^{th} embodiment, the problem is solved by a use of a polypeptide comprising a Ses iPR-10 allergen from sesame characterized by SEQ ID NO1 or a variant thereof, the polypeptide, carrier or kit according to any of claims 1 to 6 for detecting an antibody binding specifically to a Ses iPR-10 allergen from sesame characterized by SEQ ID NO1.

In an 8^{th} embodiment, the problem is solved by a use of a polypeptide comprising a Ses iPR-10 allergen from sesame characterized by SEQ ID NO1 or an antibody binding specifically to a Ses iPR-10 allergen from sesame characterized by SEQ ID NO1 or the carrier according to any of claims 3, 4 or 6 for the manufacture of a diagnostic kit.

In a 9^{th} embodiment, the problem is solved by a use of a polypeptide comprising a Ses iPR-10 allergen from sesame characterized by SEQ ID NO1 or an antibody binding specifically to a Ses iPR-10 allergen from sesame characterized by SEQ ID NO1 or the carrier according to any of claims 3, 4 or 6 for diagnosing or aiding in the diagnosis of an allergy.

In a preferred embodiment, the antibody is an IgE or IgG class antibody.

In a preferred embodiment, the antibody is detected using a method from the group comprising immunodiffusion, immunoelectrophoresis, light scattering, agglutination, labeled immunoassays, preferably from the group comprising radiolabeled immunoassays, enzyme immunoassays such as colorimetric assays, chemiluminescence, preferably electrochemiluminescence immunoassays and immunofluorescence immunoassays.

In a 9^{th} aspect, the problem is solved by an enriched, isolated, diluted or purified antibody, preferably IgE or IgG class antibody or recombinant antibody binding specifically to a polypeptide a Ses iPR-10 allergen from sesame characterized by SEQ ID NO1, wherein the antibody is preferably in a solution comprising an artificial buffer or a natural buffer at a non-physiological concentration. The problem may be solved by an antibody in a solution comprising an artificial buffer and/or a physiological buffer at non-physiological concentrations, preferably a concentration higher or lower, preferably lower than at physiological conditions. Preferably, the antibody is in a diluted sample from a patient.

The present invention is based on the inventors' surprising finding that antibodies exist in samples from patients suffering from an allergy which bind to Ses iPR-10 allergens from sesame characterized by SEQ ID NO1.The inventors have found that such antibodies do not exist in healthy patients and can be used to diagnose or aid in the diagnosis of a sesame allergy or a plant PR-10 allergy.

According to the present invention a polypeptide comprising a sesame Ses iPR-10 allergen from sesame characterized by SEQ ID NO1or a variant thereof is provided. In a preferred embodiment, the term "Ses iPR-10 allergen from sesame characterized by SEQ ID NO1" refers any polypeptide which is expressed by a plant from the order *Lamiales,* preferably *Sesamum sp.,* more preferably *Sesamum indicum* and has a sequence which comprises SEQ ID NO1, preferably SEQ ID NO17, more preferably SEQ ID NO18 or a variant thereof. Preferably, the Ses iPR-10 allergen from sesame characterized by SEQ ID NO1 comprises 170 or less amino acids when any signal sequences are removed or discounted. In a preferred embodiment, the Ses iPR-10 allergen from sesame characterized by SEQ ID NO1 comprises SEQ ID NO17, more preferably SEQ ID N018 and is most preferably selected from the group comprising SEQ ID NO2 (Ses iPR-10-2), SEQ ID NO3 (Ses iPR-10-3), SEQ ID NO4 (Ses iPR-10-4), SEQ ID NO5 (Ses iPR-10-5) and SEQ ID NO6 (Ses iPR-10-6). In a preferred embodiment, the Ses iPR-10 allergen has SEQ ID NO28 and is preferably selected from the group comprising SEQ ID NO2 (Ses iPR-10-2), SEQ ID NO5 (Ses iPR-10-5) and SEQ ID NO6 (Ses iPR-10-6) and variants thereof.

In a preferred embodiment, the Ses iPR-10 allergen from sesame characterized by SEQ ID NO1 comprises SEQ ID NO2 or a variant thereof. In a preferred embodiment, it comprises SEQ ID NO3 or a variant thereof. In a preferred embodiment, it comprises SEQ ID NO4 or a variant thereof. In a preferred embodiment, it comprises SEQ ID NO5 or a variant thereof. In a preferred embodiment, it comprises SEQ ID NO6 or a variant thereof. In a preferred embodiment, the term "a plant PR-10 allergy", as used herein, refers to the display of allergic symptoms, preferably an allergy, to a variety of plant pathogenesis related (PR) proteins. These proteins are induced by the plants as a defense response system in stress conditions like microbial and insect infections, wounding, expose to harsh chemicals and atmospheric conditions (Sinha *et al.,* The Scientific World Journal, 2015, ID 543195). Examples include Bet v 1, Cor a 1, Mal d 1, Api g 1, Gly m 4, Jug r 5 and Pru av 1. In a preferred embodiment, a patient suffering from a plant PR-10 allergy shows an IgE reaction to at least 1, 2, 3, 4, 5, 6, 7, 8, 9 or 10 different PR proteins.

The invention relates to a diagnostically useful carrier, which is preferably a solid phase carrier for contacting a means for specifically binding an antibody associated with said carrier, preferably a polypeptide comprising a sesame Ses iPR-10 allergen from sesame characterized by SEQ ID NO1 or a variant thereof with a bodily fluid sample from a subject, preferably a mammalian subject, more preferably a human subject.

In a preferred embodiment, the carrier comprises, in addition, one or more means for specifically binding an antibody, preferably one or more, more preferably two or more, more preferably three or more such means, each of them capable of specifically capturing a different antibody, preferably from the group comprising an antibody to sesame extract, an antibody to Q9AUD1 (Ses i 1), an antibody to Q9XHP1 (Ses i 2), an antibody to Q9AUD0 (Ses i 3), an antibody to Q9XHP0 (Ses i 6), an antibody to Q9AUD2 (Ses i 7), an antibody to Bet v 1, an antibody to Cor a 1, and antibody to Mal d 1, an antibody to Gly m 4, an antibody to Jug r 5, and an antibody to Pru av 1 and an antibody to a sesame LTP characterized by SEQ ID NO11 from European patent application EP21161331.0, preferably from the group comprising SEQ ID NO1, SEQ ID NO2, SEQ ID NO3, SEQ ID NO4 and SEQ ID NO5, each from EP21161331.0. Preferably such means is the polypeptide to which the antibody to be bound and/or detected binds or variant thereof. For example, a polypeptide comprising Ses i 1 or a variant thereof is a means for specifically binding or capturing an antibody to Ses i 1. In a preferred embodiment, the polypeptide comprising a Ses iPR-10 allergen from sesame characterized by SEQ ID NO1, an antibody to said polypeptide, a kit or a carrier according to the present invention may be used to increase the sensitivity and/or specificity of a diagnosis based on the detection of one or more antibodies from the aforementioned list of antibodies, preferably an antibody reactive with sesame extract or an antibody to a polypeptide having the sequence represented by Q9AUD1 (Ses i 1).

The means for specifically binding or capable of specifically capturing may be the polypeptide to which is antibody to be bound or captured binds, or a variant of said polypeptide, for example a polypeptide having SEQ ID NO1 or variant thereof if an antibody to SEQ ID NO1 is to be detected.

Throughout this application, any data base protein sequence code refers to the version available online at the earliest priority or filing date. Said one or more means is preferably immobilized on said carrier. In a preferred embodiment, the means for specifically binding an antibody is a polypeptide comprising or consisting of an antigen to which the antibody to be captured or detected binds specifically or a variant thereof. The polypeptide may be a linear peptide or a folded polypeptide, the latter preferably a variant adopting essentially the same fold as the native protein as may be determined by CD spectroscopy. In a preferred embodiment, the polypeptide comprises an epitope to the antibody to be captured or detected of at least 7, preferably 10, more preferably 15 amino acids. Said means, together with the insoluble carrier to which it is attached, may be separated from a reaction mixture, wherein it is contacted with a sample, in a straightforward manner, for example by filtration, application of a magnetic field, centrifugation or decanting.

In a preferred embodiment, the carrier is a line blot (Raoult, D., and Dasch, G. A. (1989), the line blot: an immunoassay for monoclonal and other antibodies. Its application to the serotyping of gram-negative bacteria. J. Immunol. Methods, 125 (1-2), 57-65; WO2013041540). In a preferred embodiment, the term "line blot", as used herein refers to a test strip, more preferably membrane-based, that has been coated with one or more means for specifically binding an antibody. If two or more means are used, they are preferably spatially separated on the carrier. Preferably, the width of the bands is at least 30, more preferably 40, 50, 60, 70 or 80% the width of the test strip. The line blot may comprise one or more control bands for confirming (a) that it has been contacted with sample sufficiently long and under adequate conditions, in particular in the presence of human serum, and/or (b) that essential reagents were present, in particular a secondary antibody and/or (c) a CCD control. A cut off band indicating whether a signal from another band is sufficiently strong to represent a positive result may also be included.

According to the present invention, a medical or diagnostic device such as the diagnostically useful carrier may be prepared by expressing a recombinant variant of a sesame Ses iPR-10 characterized by SEQ NO 1 comprising an affinity tag, optionally with an artificial linker which may include a protease cleavage site, in a cell such as a eukaryotic or prokaryotic cell, contacting the expressed variant with a ligand binding specifically to the affinity tag, which ligand is immobilized on a solid phase, washing the solid phase such that non-specifically bound material from the cell is removed and eluting the expressed variant from the solid phase, preferably by adding an excess of non-immobilized ligand. The variant may then be immobilized on the device. Optionally, the affinity tag may be removed by contacting the variant with a protease, preferably a protease recognizing the protease cleavage site such as the commercially available precission protease, before the immobilization. The affinity tag may be selected from the group of tags comprising 18A, ACP, Aldehyd, Avi, BCCP, Calmodulin, Chitin binding protein, E-Tag, ELK16, FLAG, flash, poly glutamate, poly aspartate, GST, GFP, HA, Isope, maltose binding protein, myc, nus, NE, ProtA, ProtC, Rhodopsin Epitope Tag, Tho1d4, S-Tag, SnoopTag, SpyTag, SofTag, Streptavidin, Strep-tag II, T7 Epitope Tag, TAP, TC, Thioredoxin, Ty, V5, VSV and Xpress Tag. Useful proteases include, but are not limited to TEV, Thrombin, Factor Xa or Enteropeptidase. Suitable linkers are part of vectors, for example pET vector series (Novagen).

In a preferred embodiment, the polypeptide comprising a Ses iPR-10 allergen from sesame characterized by SEQ NO1 or a variant thereof is immobilized on a carrier. In a preferred embodiment, the term "coated", as used herein and used interchangeably with "immobilized", means that the polypeptide is immobilized on the surface of a carrier. Any immobilization may be in a reversible or irreversible manner. For example, the immobilization is reversible if a polypeptide to be immobilized interacts with the carrier via ionic interactions which may be masked by addition of a high concentration of salt or if the polypeptide is bound via a cleavable covalent bond. By contrast, the immobilization is irreversible if such polypeptide is tethered to the carrier via a covalent bond that cannot be cleaved in aqueous solution under physiological conditions. For example, highly reactive esters such as N-hydroxysuccinimide may be used to immobilize a polypeptide irreversibly on a bead. The polypeptide may be indirectly immobilized, for example by immobilizing an antibody or other entity having affinity to the polypeptide, followed by addition of the polypeptide and formation of a polypeptide-antibody complex. The antibody captured may be detected using a labeled secondary antibody or a labeled means for specifically capturing the antibody to be detected. Immobilized polypeptides or other molecules may be provided for the methods, uses and products according to the invention in an immobilized form or in a form configured for immobilization, in particular with an affinity tag binding specifically to a ligand coated on a carrier on which the polypeptide is to be immobilized. For example, the carrier may be coated with streptavidin and the immobilized polypeptide may carry a biotin label or *vice versa.* Methods for immobilizing are described in Wong, S. S., Chemistry of Protein Conjugation and CrossLinking, 1991, CRC Press, and in Rosenberg, I. M., Protein Analysis and Purification, Second Edition, Springer, 2005, and in Thermo Scientific Pierce Antibody Production and Purification Technical Handbook, online version 2020.

In a preferred embodiment, a CCD control, which may be on the carrier, allows the identification of false positive results due to binding of IgE or similar antibodies to glycoprotein epitopes such as alpha-1,3-fucose on N-glycans (Altmann, F. (2016) Coping with cross-reactive carbohydrate determinants in allergy diagnosis, Allergo J Int 25(4), 98-105). This may be an additional line in a line blot or well in a microtiter plate, separate bead or other carrier or spatially separate part thereof comprising glycoprotein epitopes that, if negative, shows that there is no false negative reactivity in other bands. A multitude of line blots are commercially available, for example from EUROIMMUN Medizinische Labordiagnostika AG, Lübeck, Germany. In a preferred embodiment, the method according to the present invention comprises the step detecting the presence or absence of an antibody that binds to cross-reactive carbohydrate epitopes and so leads to false positive results. In another preferred embodiment, the method comprises treating a sample with CCD absorbent to remove such an antibody, and such an absorbent (ZD 3001-0101, EUROIMMUN) may be part of a kit according to the present invention.

In another preferred embodiment, the diagnostically useful carrier is a bead. Various beads for numerous applications are commercially available, mainly based on carbohydrate, for example sepharose or agarose, or plastic. They contain active or activatable chemical groups such as a carboxyl or ester group, which can be utilized for the immobilization of a means for specifically binding an antibody. Preferably, the beads are beads having an average diameter of from 0.1 µm to 10 µm, from 0.5 µm to 8 µm, from 0.75 µm to 7 µm or from 1 µm to 6 µm. The beads can be coated with the means for specifically capturing an antibody directly or *via* affinity ligands, for example biotin or glutathione and streptavidin and GST, respectively. For example, the bead may be coated with biotin or glutathione and the means for specifically binding an antibody or any polypeptide may be fused with streptavidin or glutathione-S-transferase or a variant thereof, respectively. Preferably, the bead is provided in the form of an aqueous suspension having a bead content of from 10 to 90%, preferably from 20 to 80%, preferably from 30 to 70%, more preferably from 40 to 60% (w/w). In a preferred embodiment, the bead is coated with a first detectable marker, for example a fluorescent dye. Chemiluminescence, preferably electrochemiluminescence or fluorescence is a preferred method of detection if the carrier is a bead.

If more than one type of bead on which a distinct means for binding specifically an antibody to be detected is coated is used, each type may have a different type of detectable marker to make the types of beads distinguishable, for example for identifying the type of bead and bound antibody in flow cytometry. If the presence of an antibody binding specifically to a means is detected using a fluorescent label, the first detectable label, if also a fluorescent label, is chosen such that both can be distinguished, for example using separate lasers such as a green and a red laser for the detection of the label.

In a preferred embodiment, a detectable label is used to detect an antibody according to the present invention, which is a label that may be used to distinguish a population of molecules from others using biophysical detection methods. It is preferably selected from the group comprising a fluorescent, a radioactive, a chemiluminescent label, a heavy metal such as gold label, a nanoparticle, a bead or an enzymatically active label, preferably one catalyzing a colorimetric reaction. In a preferred embodiment, a fluorescent label is selected from the group comprising Alexa dyes, FITC, TRITC and green fluorescent protein (GFP). Iodine-125 may be used as radioactive label. In a preferred embodiment, an enzymatically active label is selected from the group comprising horseradish peroxidase, glucose oxidase, beta galactosidase, alkaline phosphatase and luciferase. The person skilled in the art is able to choose suitable labels and to attach them to proteins, nucleic acids and other molecules (Hassanzadeh L, Chen S, Veedu RN. Radiolabeling of Nucleic Acid Aptamers for Highly Sensitive Disease-Specific Molecular Imaging. Pharmaceuticals (Basel). 2018;11(4):106. Published 2018 Oct 15. doi:10.3390/ph11040106, Bioconjugate Techniques, 3rd Edition (2013) by Greg T. Hermanson, Obermaier C, Griebel A, Westermeier R. Principles of protein labeling techniques. Methods Mol Biol. 2015;1295:153-65), and a wide range of labeled molecules are commercially available.

In a particularly preferred embodiment, the beads are paramagnetic beads, which can be easily concentrated on a surface with the aid of a magnet. For this purpose, commercial paramagnetic beads usually contain a paramagnetic mineral, for example iron oxide. A multiplicity of suitable paramagnetic beads is commercially available. A bead may be labeled with a detectable label.

In another preferred embodiment, the carrier is a hydrophilic branched polymer, preferably a cellulose polymer. Such polymers which may carry chemically reactive groups for the immobilization of antigenic polypeptides are commercially available, for example the ImmunoCAP from Phadia.

In another preferred embodiment, the carrier is a microtiter plate comprising at least 8 wells that may be used for ELISA. At least one of the wells is coated with the one or more means for specifically binding an antibody, either directly or indirectly. Two or more wells may be coated with a calibrator each.

According to the present invention, for ELISA, but also other assay formats products and uses, at least 2, 3, preferably 4, more preferably 5 calibrators are provided. In a preferred embodiment, a calibrator comprises an antibody binding specifically to said one or more means, preferably at a defined concentration and may be used to set up a calibration curve for semi-quantitative analysis see Wild, The Immunoassay Handbook, 3rd Edition, 2005, Elsevier, Chapter 9. Preferably it is an antibody binding specifically to a sesame Ses iPR-10 allergen from sesame characterized by SEQ ID NO1, but it could also be an antibody or similar reagent which binds specifically to the same means for capturing an antibody and/or the same means for detecting an antibody as the antibody to be detected does, preferably binding specifically to a sesame Ses iPR-10 allergen from sesame characterized by SEQ ID NO1 and to a secondary antibody binding specifically to human IgE or IgG class antibodies. For example, a chimeric antibody comprising the same constant region as the antibody to be detected does, for example comprise the constant region of a human IgE or IgG antibody, while the variable region may be or may include parts from a recombinant antibody binding specifically to a sesame Ses iPR-10 allergen from sesame characterized by SEQ ID NO1. See Hamilton RG. Application of engineered chimeric antibodies to the calibration of human antibody standards. Ann Biol Clin (Paris). 1991;49(4):242-8. PMID: 1928840. A set of calibrators comprises calibrators that comprise said antibody at known concentrations, which differ from each other. They are typically chosen such that they cover a significant part of the detection range for example, two calibrators may be provided at concentration ratios of at least 1:2, 1:5, 1:10, 1:50, 1:100, 1:200, 1:500 and 1:1000. A calibrator reflecting the absence of the antibody to be detected in a sample may also be included. When the inventive method is carried out, the calibrators may be processed and developed in parallel to the samples or before the samples are processed and developed. In a preferred embodiment, a calibration curve is stored and/or may have to be repeated only at periodic intervals.

In a preferred embodiment, a label is a chemical group which may be detected, preferably from the group comprising an enzymatically active label, for example a label having horse radish peroxidase activity or alkaline phosphatase activity, a chemiluminescent label, for example a molecule capable of generating a chemiluminescent signal such as an acridinium ester or luminol, a radioactive label, for example iodine-125, a spin label and a fluorescent label, for example FITC.

In another preferred embodiment, the carrier is a microarray. In a preferred embodiment, the term "microarray", as used herein, refers to a chip spotted with a variety of spatially separate allergenic polypeptides, among them one or more than one polypeptide according to the present invention or a variant thereof, preferably at least 5, 10, 20, preferably at least 30, 40, 50, 80 or 100 polypeptides.

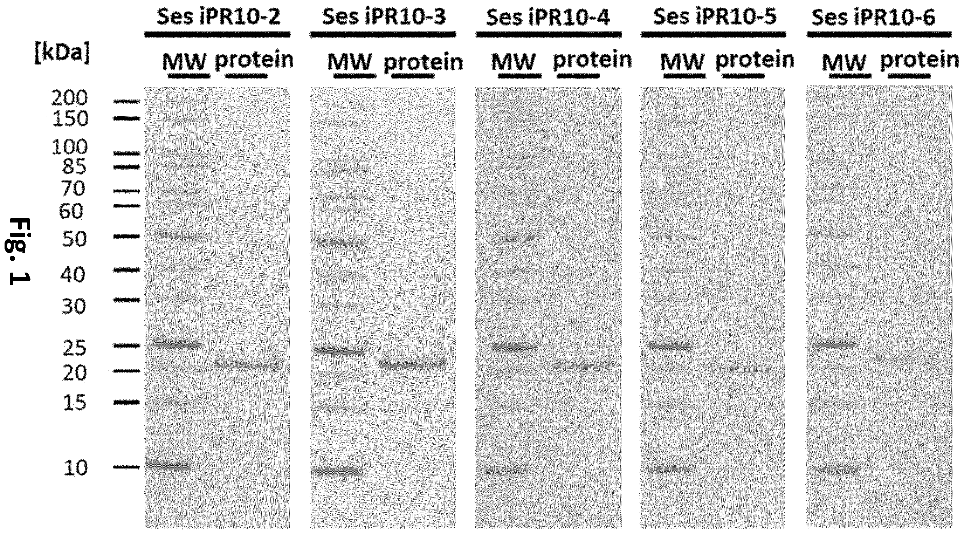

In a preferred embodiment, the term "a means for detecting an antibody", as used herein, refers to a reagent that makes an antibody detectable which is part of a complex comprising an antigen such as a sesame Ses iPR-10 allergen from sesame characterized by SEQ ID NO1 or a variant thereof immobilized on a carrier. In a preferred embodiment, the means is a molecule which binds specifically to all antibodies which have the same immunoglobulin class as the antibody to be detected, preferably IgE or IgG, more preferably a secondary antibody. In a preferred embodiment, the means is a molecule which binds specifically all antibodies from the subject to be diagnosed, preferably a mammal, more preferably a human. Such a means may be a mixture of secondary antibodies or a polypeptide comprising the Ses iPR-10 allergen from sesame characterized by SEQ ID NO1 to which the antibody to be detected binds, or a variant thereof. For example, the secondary antibody could be used in an immunometric assay, and the polypeptide could be used in a capture bridge assay (Wild, The Immunoassay Handbook, 3rd Edition, 2005, Elsevier, Figs. 1.2 and 1.42, respectively). In another preferred embodiment, the means is a Ses iPR-10 allergen from sesame characterized by SEQ ID NO1 or a variant thereof. It may bind to an antibody to be detected, which is or will be immobilized such that at least one of its antigen binding sites is unoccupied and accessible for binding. For example, the antibody could be immobilized via its constant region or via one of its more than one binding sites on its variable regions. In a preferred embodiment, the means carries a detectable label. In a preferred embodiment, the means for detecting an antibody can be used to detect one or more mammalian, preferably primate, more preferably human antibodies. In accordance with the present invention, the term "secondary antibody" in its broadest sense is to be understood to refer to any kind of "binding moiety", preferably binding protein, capable of specific binding to an antibody or a fragment thereof, preferably IgE and/or IgG class antibody, such as a constant domain of a particular Ig class of a selected species, preferably human. Non-limiting examples of binding moieties include antibodies, for example antibodies immunologically or genetically derived from any species, for example human, chicken, camel, llama, lamprey, shark, goat, rodent, cow, dog, rabbit, etc., antibody fragments, domains or parts thereof, for example Fab, Fab', F(ab')₂, scFab, Fv, scFv, VH, VHH, VL, VLRs, and the like, diabodies, monoclonal antibodies (mAbs), polyclonal antibodies (pAbs), mAbdAbs, phage display-derived binders, affibodies, heteroconjugate antibodies, bispecific antibodies, evibodies, lipocalins, anticalins, affibodies, avimers, maxibodies, nanobodies, heat shock proteins such as GroEL and GroES, trans-bodies, DARPins, aptamers, C-type lectin domains such as tetranectins; human γ-crystallin and human ubiquitin-derived binders such as affilins, PDZ domain-derived binders, scorpion toxin and/or Kunitz-type domain binders, fibronectin-derived binders such as adnectins, receptors, ligands, lectins, avidin, streptavidin, biotin, including derivatives and/or combinations thereof such as bi-/multi-specific formats formed from two or more of these binding molecules. Various antibody-derived and alternative (i.e. non-antibody) binding protein scaffolds including methods of generation thereof are known in the art (e.g. reviewed in Chiu ML et al., Antibodies (Basel), (2019);8(4):55; Simeon R. & Chen Z., Protein Cell. (2018);9(1):3-14; and Chapter 7 - Non-Antibody Scaffolds from Handbook of Therapeutic Antibodies (2007) edited by Stefan Dübel. Direct reference is made to the description of EP21158065.9, wherein these molecules are defined and their use and generation outlined.

In a preferred embodiment, a secondary antibody is an antibody binding specifically to all antibodies from one or more immunoglobulin classes, preferably one or more or all from the group comprising human IgA, human IgM, human IgE and human IgG or the, more preferably a human immunoglobulin class such as IgG or IgE. Secondary antibodies typically recognize the constant domain of said class, but may also recognize other epitopes shared by antibodies from the class of interest, for example a conformational epitope across the 3D structure. A wide range of them is commercially available, for example from Thermo Fisher. It may be a monoclonal or a polyclonal antibody. In a preferred embodiment, the term "recognized", as used herein, means that the secondary antibody binds specifically to the antibody or antibodies to be detected. A secondary antibody may bind specifically to one or to more than one immunoglobulin class. This may be achieved by using as a secondary antibody to the class, preferably to IgG class antibodies, a mixture comprising an antibody binding specifically to each immunoglobulin or a single antibody which reacts with all immunoglobulin classes of interest. The use of secondary antibodies is explained in Kruger, N. J., Detection of Polypeptides on Blots Using Secondary Antibodies, in The Protein Protocols Handbook (ed. J. M. Walker), page 967, volume 1996, Springer. Briefly, such secondary antibodies may be generated by immunizing a laboratory animal with the antibody to be recognized or a mixture of the antibodies to be recognized.

In a preferred embodiment, the term "a means for capturing an antibody", as used herein, is a means that may be used to immobilize an antibody on a solid phase, preferably all antibodies from an immunoglobulin class or an antibody binding specifically to a Ses iPR-10 allergen from sesame characterized by SEQ ID NO1 or a variant thereof. The means can be a secondary antibody, aptamer or other protein immobilized on the solid phase, which binds specifically to all antibodies having an immunoglobulin class selected from the group comprising IgA, IgM, IgG and IgE, preferably IgG and/or IgE. The immunoglobulin class is more preferably a human immunoglobulin class. The means can be a Ses iPR-10 allergen from sesame characterized by SEQ ID NO1 or a variant thereof immobilized on the solid phase such that it only captures an antibody binding specifically to a Ses iPR-10 allergen from sesame characterized by SEQ ID NO1. Typically, the means does not carry a detectable label, by contrast to the means for detecting an antibody.

The sample from a subject preferably used to practice the present invention comprises antibodies, also referred to as immunoglobulins. Typically the sample is a bodily fluid comprising a representative set of the entirety of the subject's immunoglobulins. However, the sample, once provided, may be subjected to further processing which may include fractionation, centrifugation, enriching or isolating the entirety of immunoglobulins or any immunoglobulin class of the subject, preferably IgE and/or IgG, which may affect the relative distribution of immunoglobulins of the various classes. The sample may be selected from the group comprising whole-blood, serum, plasma, and saliva and is preferably serum. In a most preferred embodiment, the sample comprises IgE class antibodies. In a more preferred embodiment, the sample comprises a representative set of the subject's antibodies from classes IgA, IgG, IgM, and IgE, preferably IgG and IgE, more preferably IgG1, IgG4 and IgE, wherein, most preferably, the ratio of antibodies to different antigens is essentially unaltered compared to the ratio in the sample as obtained from the subject. The sample is preferably from a mammal, more preferably a primate, most preferably a human.

The teachings of the present invention may not only be carried out using polypeptides having the exact sequences referred to in this application explicitly, for example by function, name, sequence or accession number, or implicitly, but also using variants of such polypeptides.

In a preferred embodiment, the term "variant", as used herein, may refer to at least one fragment of the full-length sequence referred to, more specifically one or more amino acid which is, relative to the full-length sequence, truncated at one or both termini by one or more amino acids. Such a fragment comprises or encodes a peptide having at least 6, 7, 8, 9, 10, 15, 25, 50, 60, 70, 80, 90 or 100 successive amino acids of the original sequence or a variant thereof, preferably at least 8. In a more preferred embodiment, the total length of the variant may be at least 25, 30, 40, 50, 60, 70, 80, 90, 100, 120, 130, 140 or 150 or more amino acids. In another more preferred embodiment, the total length of the variant may be no more than 25, 30, 40, 50, 60, 70, 80, 90, 100, 150, 200 or 250 or more amino acids.

In another preferred embodiment, the term "variant" relates not only to at least one fragment, but also a polypeptide or a fragment thereof comprising amino acid sequences that are at least 40, 50, 60, 70, 75, 80, 85, 90, 92, 94, 95, 96, 97, 98 or 99% identical to the reference amino acid sequence referred to or the fragment thereof, wherein amino acids other than those essential for the biological activity, for example the ability to bind specifically to an antibody to be detected, or the fold or structure of the polypeptide are deleted or substituted and/or one or more such essential amino acids are replaced in a conservative manner and/or amino acids are added or deleted such that the biological activity of the polypeptide is at least partially preserved. The state of the art comprises various methods that may be used to align two given nucleic acid or amino acid sequences and to calculate the degree of identity, see for example Arthur Lesk (2008), Introduction to bioinformatics, Oxford University Press, 2008, 3rd edition. In a preferred embodiment, the ClustalW software (Larkin, M. A., Blackshields, G., Brown, N. P., Chenna, R., McGettigan, P. A., McWilliam, H., Valentin, F., Wallace, I. M., Wilm, A., Lopez, R., Thompson, J. D., Gibson, T. J., Higgins, D. G. (2007): Clustal W and Clustal X version 2.0. Bioinformatics, 23, 2947-2948) is used applying default settings.

In a preferred embodiment, variants may, in addition, comprise chemical modifications, for example labels such as isotopic labels or detectable labels or covalent modifications such as glycosylation, phosphorylation, acetylation, decarboxylation, citrullination, hydroxylation and the like. The person skilled in the art is familiar with methods for the modification of polypeptides. Moreover, variants may also be generated by way of fusion with other known polypeptides or variants thereof, for example artificial linkers, preferably not derived from a *Sesame* polypeptide, affinity tags, other antigens and the like. In particular, the variant may comprise biotin or streptavidin.

The variant of the polypeptide, in particular a Ses iPR-10 allergen from sesame characterized by SEQ ID NO1, has biological activity. In a preferred embodiment such biological activity is the ability to bind specifically to the respective antibody to be detected, more preferably taken from the sample of a patient suffering from a disease or allergy associated with the presence of such an antibody, in particular an antibody to a Ses iPR-10 allergen from sesame characterized by SEQ ID NO1. In a preferred embodiment it comprises an epitope having the ability to bind specifically to the respective antibody, preferably from a sample from a patient suffering from a plant allergy, more preferably sesame allergy or a plant PR-10 allergy, associated with the presence of such an antibody, in particular an antibody to a Ses iPR-10 allergen from sesame characterized by SEQ ID NO1. The epitope may comprise at least 6, 7, 8, 9, 10, 20, 30 or 40 amino acids from the Ses iPR-10 allergen from sesame characterized by SEQ ID NO1. A folded epitope is particularly preferred. Exemplary variants of SEQ iPR-10 allergens include SEQ ID NO7 to SEQ ID NO to 11, SEQ ID NO19 to SEQ ID NO21 and SEQ ID NO25 to SEQ ID NO27.

When designing variants, the person skilled in the art may consider epitopes contributing to the biological activity.

In a preferred embodiment, a line blot assay comprising the variant as a means for specifically capturing an antibody, preferably as described in the examples, may be used to test whether the variant has such activity. The variant may also comprise N-terminal and/or C-terminal peptide or polypeptide fusions as long as these do not have specific binding activity to antigenic sequence parts and/or do not bind to other sequence parts, thus masking the antigenic sequence such that its binding characteristics are significantly altered and/or it is no longer capable of binding to the antibody. Exemplary fusion variants include SEQ ID NO7, SEQ ID NO8, SEQ ID NO9, SEQ ID NO10 and SEQ ID NO11.

The polypeptide may be provided in any form and at any degree of purification, from tissues, fluids or cells comprising said polypeptide in an endogenous form, more preferably cells overexpressing the polypeptide, crude or enriched lysates of such cells, to purified and/or isolated polypeptides which may be essentially pure. In a preferred embodiment, the polypeptide is a native polypeptide, wherein the term "native polypeptide", as used herein, refers to a folded polypeptide, more preferably to a folded polypeptide purified from cells, more preferably from prokaryotic or eukaryotic, preferably mammalian cells, more preferably plant cells. In another preferred embodiment, the polypeptide is purified from sesame or processed products made from sesame. A glycosylated form of the polypeptide may be used. In a preferred embodiment, the term "purified", as used herein, means that the polypeptide or any other purified reagent or molecule has been subjected to one or more method steps that effect a purification, preferably from the group comprising chromatography, more preferably from the group comprising size exclusion, affinity, hydrophobic interaction and ion exchange chromatography, more preferably affinity chromatography using an affinity tag such as a GST, MPB or His tag.

In a preferred embodiment, the polypeptide is used in a non-reduced state. This may be accomplished by purifying the polypeptide under non-reducing conditions or by oxidizing the polypeptide following the purification, for example by exposure to air in the absence of reducing agents such as DTT.

According to the present invention, the polypeptide may be a recombinant protein, wherein the term "recombinant", as used herein, refers to a polypeptide produced using genetic engineering approaches at any stage of the production process, for example by fusing a nucleic acid encoding the polypeptide to a strong promoter for overexpression in cells or tissues or by engineering the sequence of the polypeptide itself. The person skilled in the art is familiar with methods for engineering nucleic acids and polypeptides encoded (for example, described in Sambrook, J., Fritsch, E. F. and Maniatis, T. (1989), Molecular Cloning, CSH or in Brown T. A. (1986), Gene Cloning - an introduction, Chapman & Hall) and for producing and purifying native or recombinant polypeptides (for example Handbooks "Strategies for Protein Purification", "Antibody Purification", published by GE Healthcare Life Sciences, and in Burgess, R. R., Deutscher, M. P. (2009): Guide to Protein Purification). In another preferred embodiment, the polypeptide is an isolated polypeptide, wherein the term "isolated" means that the polypeptide has been enriched compared to its state upon production or as part of a natural raw material such as an unprocessed sample using a biotechnological or synthetic approach and is preferably pure, *i.e*. at least 60, 70, 80, 90, 95 or 99 percent of the polypeptide in the respective liquid consists of said polypeptide as judged by SDS polyacrylamide gel electrophoresis followed by Coomassie blue staining and visual inspection. Preferably any polypeptide on a carrier used as a means to capture an antibody is pure.

The inventive teachings provide a kit, preferably for diagnosing a plant food allergy, more preferably sesame allergy or a plant PR-10 allergy. Such a kit is a container that comprises specific reagents required to practice the inventive method, in particular the diagnostically useful carrier according to the present invention, optionally in addition to one or more, preferably all reagents required to practice the inventive method. The kit may comprise a means for detecting an antibody to a sesame Ses iPR-10 allergen from sesame characterized by SEQ ID NO1. The kit may comprise a means for capturing an antibody to a sesame Ses iPR-10 allergen from sesame characterized by SEQ ID NO1. The kit may comprise a positive control, for example a recombinant antibody or antibody from a patient serum, preferably IgA, IgM, IgE or IgG, more preferably IgE and/or IgG, binding specifically to a sesame Ses iPR-10 allergen from sesame characterized by SEQ ID NO1 or to the means used to bind specifically the antibody to be detected. The recombinant antibody may be from a non-human mammal or from a bird, for example from goat, rat, chicken, mouse, rabbit, horse, sheep or non-human primate. More preferably the sample is a sample from a human. For example, a polypeptide used as a means may comprise an artificial tag, and the antibody used as a positive control may bind specifically to said tag. The kit may comprise a negative control, for example a protein which does not bind specifically to an antibody binding specifically to a Ses iPR-10 allergen from sesame characterized by SEQ ID NO1, for example bovine serum albumin. The negative control may also be a serum from a subject, preferably a human subject, who does not suffer from a plant allergy, preferably a sesame or plant PR-10 allergy. The kit may comprise one or more calibrators. The kit may comprise a suitable water-tight vessel for contacting the diagnostically useful carrier with the sample in the presence of other liquids such as a reaction buffer. For example, a line blot may be provided in or in combination with an incubation tray, or a microtiter plate may be provided. Suitable vessels are described in the state of the art, for example EP3025780 or EP3025779.

According to the present invention, specific binding between a polypeptide, in particular a Ses iPR-10 allergen from sesame characterized by SEQ ID NO1 and an antibody binding specifically to it as well as specific binding between other molecules, for example a secondary antibody and an antibody to be detected, is detected. In a preferred embodiment, the term "binding specifically", as used herein, means that the binding reaction is stronger than a binding reaction characterized by a dissociation constant of 1 × 10⁻⁵ M, more preferably 1 × 10⁻⁷ M, more preferably 1 × 10⁻⁸ M, more preferably 1 × 10⁻⁹ M, more preferably 1 × 10⁻¹⁰ M, more preferably 1 × 10⁻¹¹ M, more preferably 1 × 10⁻¹² M, as determined by surface plasmon resonance using Biacore equipment at 25 °C in PBS buffer at pH 7.

The methods or products according to the present invention may be used for diagnosing or aiding in the diagnosis an allergy, preferably a plant food allergy, more preferably a sesame allergy or a plant PR-10 allergy or for identifying a patient suffering from or having an increased risk of suffering from a plant food allergy, more preferably sesame allergy or a plant PR-10 allergy in the future. In another preferred embodiment, the allergy is characterized by a sensitization, preferably associated with clinical allergic symptoms.

In a preferred embodiment, the term "diagnosis", as used herein, is to be used in its broadest possible sense and may refer to any kind of procedure aiming to obtain information instrumental in the assessment whether a patient suffers or is likely or more likely than the average or a comparative subject, the latter preferably having similar symptoms, to suffer from a certain disease or disorder in the past, at the time of the diagnosis or in the future, to find out how the disease is progressing or is likely to progress in the future or to evaluate the responsiveness of a patient or patients in general with regard to a certain treatment, for example the administration of immunosuppressive drugs or an immunotherapeutic treatment, or to find out whether a sample is from such a patient. Such information may be used for a clinical diagnosis, but may also be obtained by an experimental and/or research laboratory for the purpose of general research, for example to determine the proportion of subjects suffering from the allergy in a patient cohort or in a population. In other words, the term "diagnosis" comprises not only diagnosing, but also prognosticating and/or monitoring the course of a disease or disorder such as an allergy, including monitoring the response of one or more patients to the administration of a drug or candidate drug or candidate vaccine, for example to determine its efficacy. While the result may be assigned to a specific patient for clinical diagnostic applications and may be communicated to a medical doctor or institution treating said patient, this is not necessarily the case for other applications, for example in diagnostics for research purposes, where it may be sufficient to assign the results to a sample from an anonymized patient. The purpose of the products, methods and uses according to the present invention may also be to determine whether a sample is from a person who suffered or is more likely to suffer from a disease in the past, at the time of the diagnosis or in the future. The purpose of the products, methods and uses according to the present invention may also be to determine whether or how many subjects in a group suffer or are likely to suffer from a disease. The group may comprise random individuals representing the overall population, a patient cohort suffering from certain symptoms or receiving a treatment. The group may comprise humans or mammals, preferably laboratory animals. In a preferred embodiment, the disease may be an allergy, preferably a plant food allergy, more preferably a sesame allergy or a plant PR-10 allergy. In another preferred embodiment, the allergy is characterized by a sensitization, preferably associated with clinical allergic symptoms, which may be associated with an antibody to a Ses iPR-10 allergen from sesame characterized by SEQ ID NO1.

In a preferred embodiment, the methods and products according to the present invention may be used for interaction studies, including determining whether a drug candidate or other compound may interfere with the binding of an antibody to a Ses iPR-10 allergen from sesame characterized by SEQ ID NO1 or may affect any downstream process or the strength of their binding to their target. In a preferred embodiment, they may be used for monitoring the immune response, more preferably the emergence and/or titer of antibodies to a Ses iPR-10 allergen from sesame characterized by SEQ ID NO1, following the administration of an immunogenic composition comprising a Ses iPR-10 allergen from sesame characterized by SEQ ID NO1 or an immunogenic variant thereof, for example to a mammal, which may be a mammal other than a human such as a laboratory animal.

The person skilled in the art will appreciate that a clinician does usually not conclude whether or not the patient suffers or is likely to suffer from a disease, condition or disorders such as an allergy solely on the basis of a single diagnostic parameter, but needs to take into account other aspects, for example the presence of other antibodies, markers, blood parameters, clinical assessment of the patient's symptoms or the results of medical imaging or other methods, in particular complementary invasive tests involving contacting the patient with one or more potential allergens, such as an SPT and/or an oral food challenge, preferably based on a polypeptide comprising a Ses iPR-10 allergen from sesame characterized by SEQ ID NO1, to arrive at a conclusive diagnosis. A basophile activation test based on the same polypeptide may be carried out.

In many cases the mere detection of the antibody, in other words determining whether or not detectable levels of the antibody are present in the sample, is sufficient for aiding in the diagnosis. If the antibody can be detected, this information will be instrumental for the clinician's diagnosis and indicates an increased likelihood that the patient suffers from an allergy. In particular, the antibody, in particular IgE detection will indicate what is referred to as a sensitization, an immunological response to a specific allergen which may confirm a suspected allergy. The patient may then be subjected to additional tests, even in vivo provocation test, such as a food challenge using sesame allergen or allergens, for example a sesame extract.

The value of a diagnostic agent or method may also reside in the possibility to rule out one allergy, thus allowing for the indirect diagnosis of another or pointing to complementary tests using additional allergens. In a preferred embodiment, the meaning of any symptoms or diseases referred to throughout this application is in line with the person skilled in the art's understanding as of the filing date or, preferably, earliest priority date of this application as evidenced by text books and scientific publications. It should be mentioned that the inventive methods or uses or products, taken alone, cannot be used to arrive at a definite, final diagnosis. The person skilled in the art is familiar with diagnostic procedures in allergy, which are detailed for example in Hamilton, R. G., Diagnosis of Human Allergic Disease, in Pediatric Allergy, 4th Edition, 2016, Elsevier.

In a preferred embodiment, the term "diagnosis" may also refer to a method or agent used to distinguish between two or more conditions associated with similar or identical symptoms, for example allergies such as sesame allergy, a plant PR-10 allergy on the one hand and another food allergy, preferably plant allergy on the other hand.

In a preferred embodiment, any information or data demonstrating the presence of absence of the antibody may be communicated to the patient or a medical doctor treating the patient orally, preferably by telephone, in a written form, preferably fax or letter, or in an electronic form *via* fax or *via* the internet, for example as an email or text message.

The present invention relates to a method comprising the step detecting in a liquid, for example a sample from a subject, the presence or absence of an antibody binding specifically to a Ses iPR-10 allergen from sesame characterized by SEQ ID NO1. Such a method may comprise the steps a) providing a liquid, preferably a patient sample, b) contacting the liquid with a carrier comprising one or more means for specifically binding the one or more antibodies to be detected under conditions compatible with the formation of a complex comprising the diagnostically useful carrier with the means and the antibody, more specifically the polypeptide comprising a Ses iPR-10 allergen from sesame characterized by SEQ ID NO1 or a variant thereof and the antibody, c) optionally isolating any said complex, for example by removing the liquid, d) optionally washing said complex, and e) detecting said complex. The method is preferably an *in vitro* method. Such a method may be used for the production of a reagent such as a positive control or calibrator for carrying out the method according to the present invention or for confirming the quality of a commercial assay.

The detection of the antibody or complex for the prognosis, diagnosis, methods or kit according to the present invention comprises the use of a method selected from the group comprising immunodiffusion techniques, immunoelectrophoretic techniques, light scattering immunoassays, agglutination techniques, labeled immunoassays such as those from the group comprising radiolabeled immunoassays, enzyme immunoassays such as colorimetric assays, chemiluminescence, preferably electrochemiluminescence immunoassays and immunofluorescence techniques. The person skilled in the art is familiar with these methods, which are also described in the state of the art, for example in Zane, H. D. (2001): Immunology - Theoretical & Practical Concepts in Laboratory Medicine, W. B. Saunders Company, in particular in Chapter 14. In a preferred embodiment, the term "presence" of an antibody, as used herein, means that the antibody is detectable using such a method, preferably a method selected from the group comprising enzyme immunoassays, more preferably colorimetric assays or chemiluminescence, preferably electrochemiluminescence immunoassays, most preferably colorimetric assays based on line blot and detection using alkaline phosphatase activity as described in the examples. In a preferred embodiment, the term "absence", as used herein, means that the antibody is not detectable using such a method, owing to its absence or presence at extremely low concentrations below the respective detection limit.

In a preferred embodiment, two or more means for binding specifically an antibody to be detected are used in particular a means for binding specifically or capturing an antibody to a Ses iPR-10 allergen from sesame characterized by SEQ ID NO1 and another allergen, which may be a means for binding specifically or capturing an antibody to another Ses iPR-10 allergen from sesame characterized by SEQ ID NO1 or to sesame extract or to Ses i 1. In a more preferred embodiment, it can be distinguished which antibody is present. This may be the case if the two or more means are spatially separated or antibodies are detected using distinguishable detection methods, for example labels comprising distinguishable fluorophores. In another preferred embodiment, it can only be determined that at least one of the two or more antibodies to be detected is present. This may be the case if a mixture of two or more means is used and it can only be determined that an antibody binds specifically to this mixture.

In many cases detecting, preferably meaning detecting the absence or presence of an antibody, optionally meaning determining whether the concentration of the antibody is above a certain threshold preferably as set by measurement using a detection method preferred according to this invention, for example ELISA or line blot, in the liquid, is sufficient for the diagnosis. If one or more antibody binding specifically to a Ses iPR-10 allergen from sesame characterized by SEQ ID NO1 can be detected, this will be information instrumental for the clinician's diagnosis and indicates an increased likelihood that the patient suffers from the allergy. The clinician will then be encouraged to follow up with more invasive tests such as oral food challenge or SPT.

In a preferred embodiment, the absence or presence of two or more antibodies is detected simultaneously, *i.e*. at the same time.

In a preferred embodiment, the absence or presence of two or more antibodies is detected in spatially separate reactions, more preferably in different reaction mixtures in separate vessels.

In a preferred embodiment, the present invention provides a use of a reagent or means or polypeptide for the detection of one or more antibodies binding specifically to a Ses iPR-10 allergen from sesame characterized by SEQ ID NO1, or of a nucleic acid encoding a Ses iPR-10 allergen from sesame characterized by SEQ ID NO1 or a variant thereof or a nucleic acid hybridizing specifically to said nucleic acid under stringent conditions or a vector or cell comprising said nucleic acid for the manufacture of a kit for the diagnosis of an allergy. In a preferred embodiment, a cell comprising said vector is provided and cultured under conditions allowing the expression of the nucleic acid, followed by isolating the expression product which may be a Ses iPR-10 allergen from sesame characterized by SEQ ID NO1 or a variant thereof, followed by use of the expression product as a means for specifically binding to an antibody in a method or product according to the present invention. More specifically, a diagnostically useful carrier may be coated with the expression product.

In another preferred embodiment, the method may be for confirming the reliability of an antibody detection assay or determining the concentration of an antibody binding specifically to a Ses iPR-10 allergen from sesame characterized by SEQ ID NO1 or a variant thereof and may involve detecting in a solution, which is not a sample from a patient, but is known or expected to comprise the antibody, preferably at a known concentration. Alternatively, the solution may be a negative control not comprising the antibody to check the background. Such method may be run in parallel with, after or before a diagnostic method.

In a preferred embodiment, the present invention provides an apparatus for analyzing a sample from a patient to detect one or more antibodies indicating an increased likelihood of an allergy, wherein the antibody is an antibody binding specifically to a Ses iPR-10 allergen from sesame characterized by SEQ ID NO1 or a variant thereof, comprising:
a) a carrier, which contains a means for capturing at least one antibody from the sample when the sample is contacted with the carrier,
b) a detectable means capable of binding to the antibody captured by the carrier when the detectable means is contacted with the carrier, particularly the detectable means is a labeled secondary antibody capable of binding to the antibody captured on the carrier,
c) optionally a means for removing any sample from the carrier and the detectable means, preferably by washing,
d) a detecting device for detecting the presence of the detectable means and converting the results into an electrical signal, and
e) optionally a means for receiving the electronical signal from the detecting device and determining if the level of the signal is indicative of an increased likelihood of a sesame allergy or a plant PR-10 allergy, by comparing with the level of signal detected in the background or an input reference value obtained with samples from healthy subjects.

The invention provides a pharmaceutical composition, preferably for immunotherapy, comprising one or more antigens selected from the group comprising a polypeptide comprising a Ses iPR-10 allergen from sesame characterized by SEQ ID NO1 or a variant thereof, optionally in combination with one or more further plant antigens, which composition is preferably suitable for administration to a subject, preferably a mammalian subject, more preferably to a human. Any polypeptides in the composition may be purified and/or recombinant. Any antigens may be fused to a carrier protein. Any allergen or variant thereof is preferably hypoallergenic. Such a pharmaceutical composition may comprise a pharmaceutically acceptable carrier. The pharmaceutical composition may, for example, be administered orally, parenterally, by inhalation spray, topically, by eyedrops, rectally, nasally, buccally, vaginally or via an implanted reservoir, wherein the term "parenterally", as used herein, comprises subcutaneous, intracutaneous, intravenous, intramuscular, intra-articular, intrasynovial, intrasternal, intrathecal, intralesional and intracranial injection or infusion techniques. The pharmaceutical composition may be provided in suitable dosage forms, for example capsules, tablets and aqueous suspensions and solutions, preferably in sterile form. It may be used in a method of treatment of a disease, preferably an allergy, which method comprises administering an effective amount of the inventive polypeptide to a subject. A hypoallergenic variant of the antigens may be used. The person skilled in the art is familiar with methods for the generation of hypoallergenic variants of known allergens. The pharmaceutical composition may be a sterilized liquid solution, which may be for injection. In a preferred embodiment, the pharmaceutical composition comprises an adjuvant, and at least one diluent, at least one glidant, at least one stabilizer and/or at least one filling agent. Suitable compositions are described in the state of the art, for example Plotkin, Orenstein and Offit, Vaccines, Elsevier Saunders, 2013 or Schijns, O'Hagan Immunopotentiators in Modern Vaccines, Elsevier Academic Press, 2006. Their use in immunotherapy is described in Varshney/Matsui, Immunotherapy for Allergic Disease, in Pediatric Allergy by Leung et al., 3rd edition, 2016, Elsevier. Treatment, ingredients and composition of such pharmaceutical compositions, useful adjuvants, carriers, the generation of hypoallergenic variants and carrier proteins are disclosed in US2021/0128781, Aglas L, Bethanis A, Chrusciel P, Stolz F, Gruen M, Jaakkola UM, Jongejan L, Yatkin E, Van Ree R. In vivo Induction of Functional Inhibitory IgG Antibodies by a Hypoallergenic Bet v 1 Variant. Front Immunol. 2020 Sep 3;11:2118, US9856296, US9040054, Orozco-Navarrete B, Kaczmarska Z, Dupeux F, et al. Structural Bases for the Allergenicity of Fra a 1.02 in Strawberry Fruits. J Agric Food Chem. 2020;68(39):10951-10961. doi:10.1021/acs.jafc.9b05714 , as well as Neudecker P, Lehmann K, Nerkamp J, et al. Mutational epitope analysis of Pru av 1 and Api g 1, the major allergens of cherry (Prunus avium) and celery (Apium graveolens): correlating IgE reactivity with three-dimensional structure. Biochem J. 2003;376(Pt 1):97-107. doi:10.1042/BJ20031057, Hofer et al.,Tackling Bet v 1 and associated food allergies with a single hybrid protein, Journal of Allergy and Clinical Immunology, 140(2) 2, 2017, 525-533 and US9844591. RNA-based allergen vaccines are described in US2017/136121.

According to the present invention, a method comprising the step enriching or isolating an antibody to a Ses iPR-10 allergen from sesame characterized by SEQ ID NO1 is provided. The person skilled in the art is familiar with methods for enriching or isolating an antibody from various sources, and exemplary procedures are described in Thermo Scientific Pierce Antibody Production and Purification Technical Handbook, Thermo Scientific. Briefly, a source of the antibody, for example a sample from a patient comprising the antibody, preferably suffering from a disease such as sesame allergy or a plant PR-10 allergy plant food allergy, or from a laboratory animal immunized with a Ses iPR-10 allergen from sesame characterized by SEQ ID NO1 or a variant thereof, or a cell overexpressing a Ses iPR-10 allergen from sesame characterized by SEQ ID NO1 or a variant thereof or a lysate of such a cell is provided. The source of the antibody in the form of a liquid solution is then contacted with a Ses iPR-10 allergen from sesame characterized by SEQ ID NO1 or a variant thereof under conditions compatible with the formation of a complex comprising the Ses iPR-10 allergen from sesame characterized by SEQ ID NO1 and the antibody, followed by separation of the liquid sample from the complex. Typically, the Ses iPR-10 allergen from sesame characterized by SEQ ID NO1 is immobilized prior to or after formation of the complex on a solid phase which may be associated with any of the carriers disclosed herein as diagnostically useful carriers or any other solid phase, for example a chromatography column, followed by washing of the complex using a washing buffer. For example, the Ses iPR-10 allergen from sesame characterized by SEQ ID NO1 may be covalently linked with an agarose bead carrying an immobilized diamino dipropylamine linker via a side chain carboxy group using a crosslinker such as EDC. This is already an isolation of the antibody, but it may optionally be dissociated from the Ses iPR-10 allergen from sesame characterized by SEQ ID NO1, for example by exposing the complex to denaturing conditions such as SDS in an electrophoretic purification step or a high salt buffer solution. Finally, the presence of the antibody, dissociated or not, may be detected by contacting it with a means for detecting an antibody. In a preferred embodiment, this method is used to prepare an antibody as a positive control, as a calibrator or for determining the concentration of the antibody in a solution, for example to prepare standardized solutions of the antibody as a control or calibrator.

According to the present invention, a polypeptide comprising a Ses iPR-10 allergen from sesame characterized by SEQ ID NO1 or an antibody binding specifically to a Ses iPR-10 allergen from sesame characterized by SEQ ID NO1, preferably from the group comprising SEQ ID NO2, SEQ ID NO3, SEQ ID NO4, SEQ ID NO5 and SEQ ID NO6, or the carrier according to the present invention may be used for the manufacture of a diagnostic kit, preferably for the diagnosis of an allergy, more preferably sesame allergy, or or a plant PR-10 allergy. For example, this may involve using the polypeptide and the antibody to coat a solid phase of a carrier, testing the quality of the carrier by performing a control reaction or calibrating with standardized components, for example a recombinant antibody rather than one from a patient sample, which may be mixed with a sample from a healthy blood donor, for example serum, packaging the components into a kit or actually performing steps of a method to obtain a result which may aid in the diagnosis.

According to the present invention, a vector comprising a nucleic acid encoding a Ses iPR-10 allergen from sesame characterized by SEQ ID NO1 or a variant is provided. Preferably, the nucleic acid is under the functional control of a promotor which allows for the constant or inducible expression of the polypeptide. Exemplary vectors include SEQ ID NO12, SEQ ID NO13, SEQ ID NO14, SEQ ID NO15, SEQ ID NO16 and variants thereof. According to the present invention, a cell, which may be prokaryotic or eukaryotic, comprising said vector is provided. The cell may be used to express the polypeptide, followed by purifying and immobilizing it on the solid phase of a device and use according to the present invention. The person skilled in the art is familiar with methods for the expression and purification of polypeptides using cells.

### Sequences:

The present invention comprises a range of novel nucleic acid and polypeptide sequences, more specifically the following, which are identical to sequences in the sequence listing. Solely as a precaution, any SEQ ID NO may refer both to sequences listed in this document and sequences in the sequence listing, in case of any unintended deviations.

"z" represents one or no amino acid, "B" a polar and/or charged amino acid, and "u" a hydrophobic amino acid including glycine, alanine and methionine, "X" one amino acid.
**SEQ ID NO1 (general Ses iPR10 antigenic consensus sequence)** 
ZZZZZZZZZZZZZZZZZZZZZZZZZZZZZZZZZZZXXXKXXXXXXGBGZXUXBUXXXBFXBXXXUBXXKXBUBXXBX
BBUXXXYBUUXGXZXUXXXUBBUXXXUBUXXXXZZXUXBUXBXXBZZZXXXXXXUXBBBUBXGKBXUXXUUXUX
BXXXZZZZZZZZZZZZZZZZZZZZZZZZZZZZZZZZZZZ
**SEQ ID NO2 (Ses iPR10-2, antigenic sequence to which antibody binds)** 
GVLTFTEEHTSAVSPSRIFKASILDANNLIPKLLPQVIKSMEFIQGTGSPGSIKQICFAEGSTLSSLKYRIDELNEETYT
YNYTVIEGDALTDKLEKITHEVKLEAAPGGGTFSKVTSTYYTKGDFALKEEDVKAGKERVLGVYKVVEAYLLNNPD
AYA
**SEQ ID NO3 (Ses iPR10-3, antigenic sequence to which antibody binds)** 
GVTSITQEFSSPVAPERLFKALILDSNNLIPKLLPQSIKSVDLLRGDGGAGSIEQVNFTETSHFKYVKHQINELDKQN
LVCKYTMIEGDALGDKLESIVYEIKFEASDINGAGCVCKMTSEYHTLGGYEVKEEEIKGGKESAMGIYKVVEAHLLE
NPHLYA
**SEQ ID NO4 (Ses iPR10-4, antigenic sequence to which antibody binds)** 
GVIKVSQSFRTKVTPSRMFKALILDSHNLCPQLMFSSIKSIDFLQGKGEPGTIKQMNFTDASPFNYVKHRIDEMDEE
KFMCKYTFIEGDALMDKLDHITYEVKFEPYGFGGCICRITSEYYTKENVEIKEEDIELGKDRAIGMYEVVEAYLLAHP
HAYN
**SEQ ID NO5 (Ses iPR10-5, antigenic sequence to which antibody binds)** 
GAITYDIEIPSSIPAAKMFKAVVLDADTLIPKIMPQAIKNVEILEGDGGVGTVKIIHFGEGSQYKSAKHRVDAIDTENLT
HSYTIVEGDALAGVLESITYHVKIVPTEDGGCICKNRSIYHTKGDVEITEEKIKEGKEKAMQMLKAIEAYLQANA
**SEQ ID NO6 (Ses iPR10-6, antigenic sequence to which antibody binds)** 
GVLNYEDELISPVPPARLFKSLVLDGDNLIPKLLPHAFKSFQILEGHGGPGTIKLVTFADGSPYKTAKHRIDEIDEEN
YIYKYSVIGGDALGEDFDSMTHGFKIEVGADGGSICKTTSTFHTKGEDHVIQEKIKAGKERAKAIFKAVETHLLAHP
HEYN
**SEQ ID NO7 (Ses iPR10-2, His tagged sequence, prescission protease cleavage site and linker, as used in examples)** 
MSHHHHHHHHLEVLFQGPSMGVLTFTEEHTSAVSPSRIFKASILDANNLIPKLLPQVIKSMEFIQGTGSPGSIKQICF
AEGSTLSSLKYRIDELNEETYTYNYTVIEGDALTDKLEKITHEVKLEAAPGGGTFSKVTSTYYTKGDFALKEEDVKA
GKERVLGVYKVVEAYLLNNPDAYA
**SEQ ID NO8 (Ses iPR10-3, His tagged sequence, prescission protease cleavage site and linker, as used in examples)** 
MSHHHHHHHHLEVLFQGPSMGVTSITQEFSSPVAPERLFKALILDSNNLIPKLLPQSIKSVDLLRGDGGAGSIEQVN
FTETSHFKYVKHQINELDKQNLVCKYTMIEGDALGDKLESIVYEIKFEASDINGAGCVCKMTSEYHTLGGYEVKEEE
IKGGKESAMGIYKVVEAHLLENPHLYA
**SEQ ID NO9 (Ses iPR10-4, His tagged sequence, prescission protease cleavage site and linker, as used in examples)** 
MSHHHHHHHHLEVLFQGPSMGVIKVSQSFRTKVTPSRMFKALILDSHNLCPQLMFSSIKSIDFLQGKGEPGTIKQM
NFTDASPFNYVKHRIDEMDEEKFMCKYTFIEGDALMDKLDHITYEVKFEPYGFGGCICRITSEYYTKENVEIKEEDIE
LGKDRAIGMYEVVEAYLLAHPHAYN
**SEQ ID NO10 (Ses iPR10-5, His tagged sequence, prescission protease cleavage site and linker, as used in examples)** 
MSHHHHHHHHLEVLFQGPSMGVLTFTEEHTSAVSPSRIFKASILDANNLIPKLLPQVIKSMEFIQGTGSPGSIKQICF
AEGSTLSSLKYRIDELNEETYTYNYTVIEGDALTDKLEKITHEVKLEAAPGGGTFSKVTSTYYTKGDFALKEEDVKA
GKERVLGVYKVVEAYLLNNPDAYA
**SEQ ID NO11 (Ses iPR10-6, His tagged sequence, prescission protease cleavage site and linker, as used in examples)** 
MSHHHHHHHHLEVLFQGPSMGVLNYEDELISPVPPARLFKSLVLDGDNLIPKLLPHAFKSFQILEGHGGPGTIKLVT
FADGSPYKTAKHRIDEIDEENYIYKYSVIGGDALGEDFDSMTHGFKIEVGADGGSICKTTSTFHTKGEDHVIQEKIKA
GKERAKAIFKAVETHLHAHPHEYN
**SEQ ID NO12 (Ses iPR10-2, vector sequence, as used in examples): only in the sequence listing**
   Only in sequence listing
**SEQ ID NO13 (Ses iPR10-3, vector sequence, as used in examples): only in the sequence listing**
   Only in sequence listing
**SEQ ID NO14 (Ses iPR10-4, vector sequence, as used in examples): only in the sequence listing**
   Only in sequence listing
**SEQ ID NO15 (Ses iPR10-5, vector sequence, as used in examples): only in the sequence listing**
   Only in sequence listing
**SEQ ID NO16 (Ses iPR10-6, vector sequence, as used in examples): only in the sequence listing**
   Only in sequence listing
**SEQ ID NO17 (more specific Ses iPR10 antigenic consensus sequence)** 
MGUXBUXXBXXBXUXUXBUFBXXUXDXBBUXPBXXZBXUKBUBXUBGBGZXUGBUXXXBFXBXSXUBXUKXBU
BXUBBBBUXXBYBUUXGBULXXXUBBUXXXUKUXXXZZZUGBUXBXBSXUXTXXXXXUXBEBUBXGKBBUXXU
UBUUEXXLXXBUZZZZ
**SEQ ID N018 (even more specific iPR10 antigenic consensus sequence)** 
MGUXBUBXBXXBXUXUXBUFKXXULDXBBLXPBUUUBXUKBUBUUBGBGXUGBUBXUBFXBXSXUBXUKXBUB
XUBBBBUXXBYBUUXGDALXXXUBBUXXXUKUXUXZZZZUGBUBBXBSXUXTXXXXXUXBEBUBXGKBBUXXUU
BUUEXXLXXBUZZZZ
**SEQ ID NO19 (sequence of variant of Ses iPR10-2, His tagged)** 
MSHHHHHHHHLEVLFQGPSMGVLTYTEEHTSAVPPARIFKASVLDADNLIPKLLPQAIKSMEILEGTGGPGTIKQIC
FAEGSTYKSAKHRIDEIDEENYTYNYTVIEGDALTDKLESITHEVKIEAAPDGGTICKVTSTYHTKGDFALKEEKIKA
GKERALGVYKAVEAYLLANPDAYA
**SEQ ID NO20 (sequence of variant of Ses iPR10-5, His tagged)** 
MSHHHHHHHHLEVLFQGPSMGVLTYDIEIPSSVPPARMFKAVVLDADNLIPKLLPQAIKSVEILEGDGGPGTIKIIHF
AEGSQYKSAKHRIDEIDEENYTYSYTVIEGDALAGVLESITHHVKIEPTEDGGCICKNTSTYHTKGDVEITEEKIKAG
KERAMQMLKAVEAYLQANP
**SEQ ID NO21 (sequence of variant of Ses iPR10-6, His tagged)** 
MSHHHHHHHHLEVLFQGPSMGVLTYEDELISPVPPARLFKALVLDADNLIPKLLPQAIKSFEILEGHGGPGTIKLITF
AEGSPYKSAKHRIDEIDEENYTYKYTVIEGDALGEDLESITHGVKIEVGADGGSICKTTSTYHTKGDDHVIEEKIKAG
KERAKAIFKAVEAYLHANPHEYN
**SEQ ID NO22 (vector sequence, used to express sequence of variant of Ses iPR10-2):**
   Only in sequence listing
**SEQ ID NO23 (vector sequence, used to express sequence of variant of Ses iPR10-5):**
   Only in sequence listing
**SEQ ID NO24 (vector sequence, used to express sequence of variant of Ses iPR10-6):**
   Only in sequence listing
**SEQ ID NO25 (sequence of variant of Ses iPR10-2)** 
MGVLTYTEEHTSAVPPARIFKASVLDADNLIPKLLPQAIKSMEILEGTGGPGTIKQICFAEGSTYKSAKHRIDEIDEEN
YTYNYTVIEGDALTDKLESITHEVKIEAAPDGGTICKVTSTYHTKGDFALKEEKIKAGKERALGVYKAVEAYLLANPD
AYA
**SEQ ID NO26 (sequence of variant of Ses iPR10-5)** 
MGVLTYDIEIPSSVPPARMFKAVVLDADNLIPKLLPQAIKSVEILEGDGGPGTIKIIHFAEGSQYKSAKHRIDEIDEEN
YTYSYTVIEGDALAGVLESITHHVKIEPTEDGGCICKNTSTYHTKGDVEITEEKIKAGKERAMQMLKAVEAYLQANP
**SEQ ID NO27 (sequence of variant of Ses iPR10-6)** 
MGVLTYEDELISPVPPARLFKALVLDADNLIPKLLPQAIKSFEILEGHGGPGTIKLITFAEGSPYKSAKHRIDEIDEENY
TYKYTVIEGDALGEDLESITHGVKIEVGADGGSICKTTSTYHTKGDDHVIEEKIKAGKERAKAIFKAVEAYLHANPHE
YN
**SEQ ID NO28 (Ses iPR10-2/5/6 consensus sequence)**

GXXXXXXEXXSXXXXXXXFKXXXLDXXXLIPKXXPXXXKXXXXXXGXGXXGXXKXXXFXXGSXXXXXKXRXDXXX
XEXXXXXYXXXXGDALXXXXXXXTXXXKXXXXXXGGXXXKXXSXXXTKGXXXXXXEXXKXGKEXXXXXXKXXEX
XLXXXXXXXX

The present invention is further illustrated by the following non-limiting examples from which further features, embodiments, aspects and advantages of the present invention may be taken.
**Fig. 1** shows gel images of the 1D-SDS-PAGE analytic from IMAC purified recombinant Ses iPR10-2, Ses iPR10-3, Ses iPR10-4, Ses iPR10-5 and Ses iPR10-6. For the analytic 4-12% gradient Bis-Tris-gels were used followed by coomassie staining. Each panel comprises a left lane with an unstained Protein Standard, Broad Range (New England Biolabs) and a right lane with purified, recombinant Ses iPR10 (reduced).

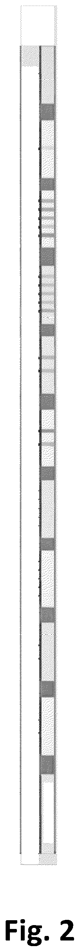

**Fig. 2** shows an exemplary line blot as produced and developed as described in example 2.

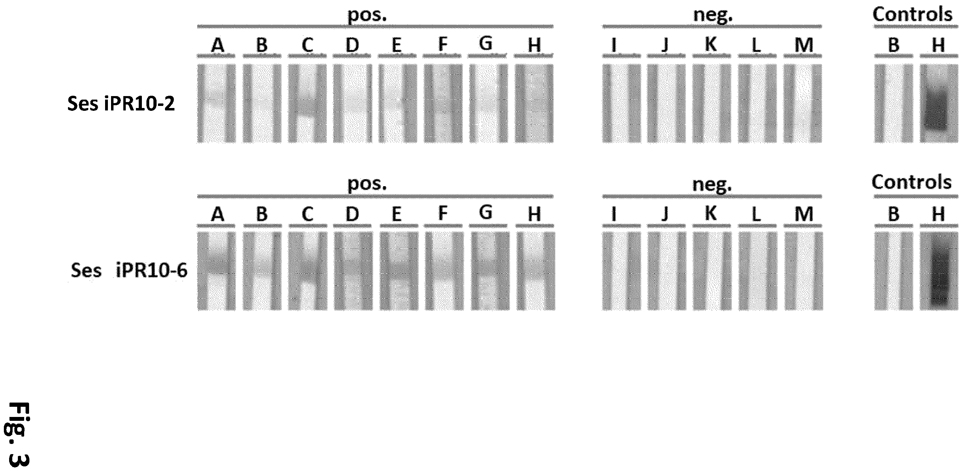

**Fig. 3** shows membrane stripe images of the Western Blot analytic from IMAC purified and 1D-SDS-PAGE separated recombinant Ses iPR10-2 and Ses iPR10-6 (reduced). For the analytic 4-12% gradient Bis-Tris-gels were used followed by electro blot transfer on a nitrocellulose membrane. As primary antibody each stripe was contacted with a diluted responding probe of the collective 1 sera or a nonresponding probe of the collective 3 sera as indicated. As secondary antibody an anti-human IgE (monoclonal) antibody conjugate (Alkaline Phosphatase) was used. Controls: for the blank (B) only the secondary antibody and for the His-Tag control (H) only an anti-poly histidine (monoclonal) antibody conjugate (Alkaline Phosphatase) was used.

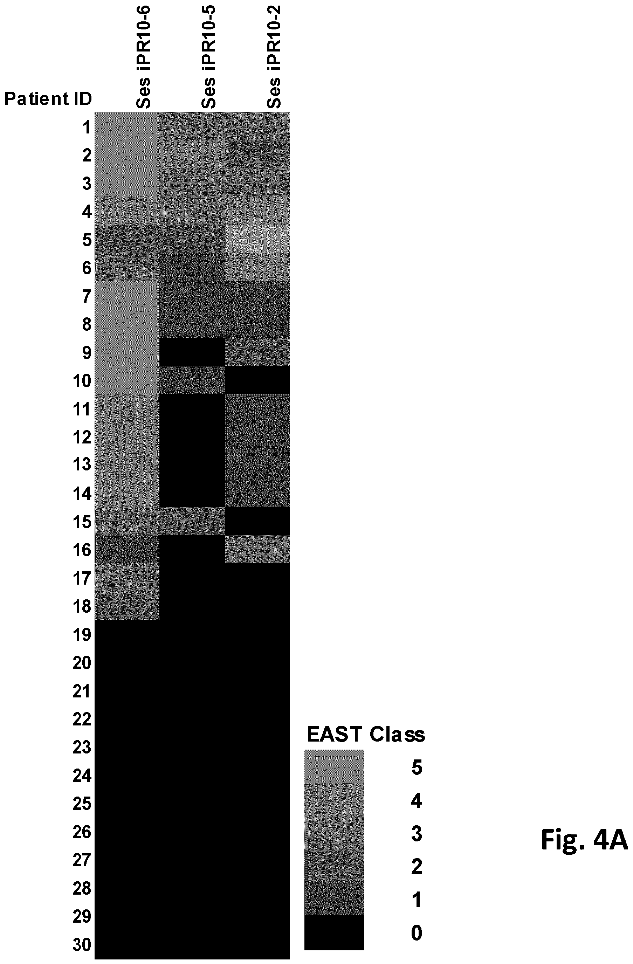

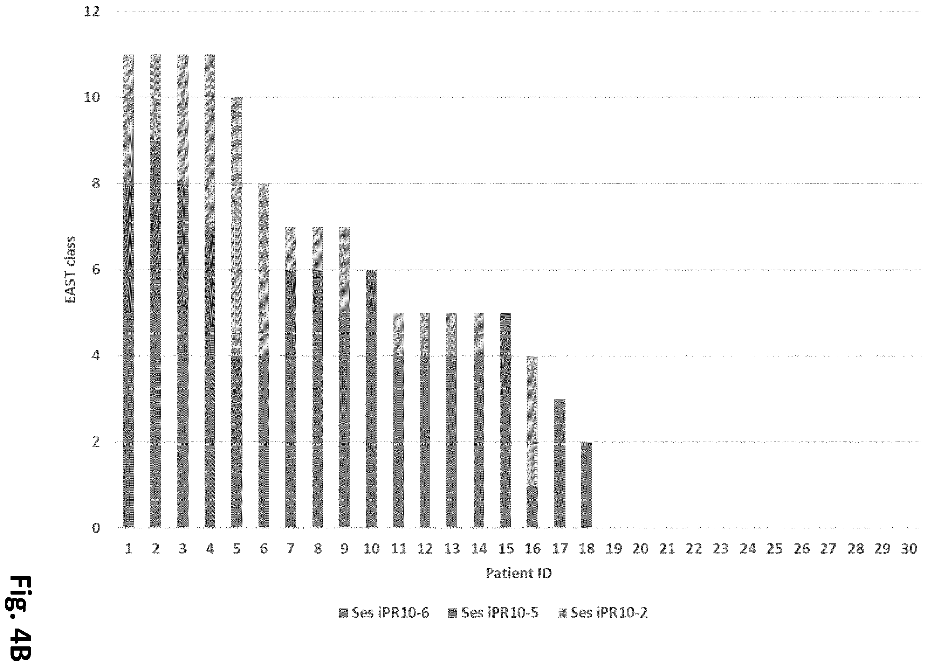

**Fig. 4** shows a heat map of IgE reactivities against iPR-10 proteins measured by immunoblot assay in 30 patient sensitized to sesame. iPR-10 proteins recognized from the same individual are shown in rows. The level of response is indicated EAST classes in different shades of green light (bright green: highest reactivity, class 5; dark green: lowest reactivity class 1; black: no reactivity).

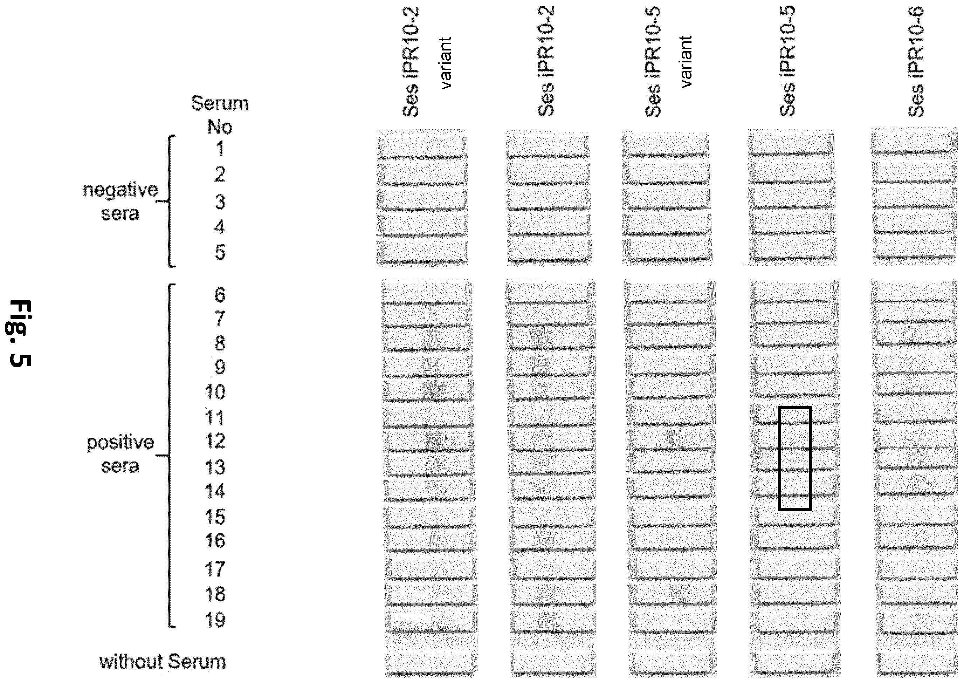

**Fig. 5** shows a western blot of Ses iPR10 and variantsreof incubated with 14 sesame positive sera and five negative sera as indicated. The back box highlights weak bands showing reactivity of Ses iPR10-5.

### Examples:

### Example 1:

Using standard molecular biology methods, each coding region of Ses iPR10-1 (SEQ ID NO: 5), Ses iPR10-2 (SEQ ID NO: 7), Ses iPR10-3 (SEQ ID NO: 18), Ses iPR10-4 (SEQ ID NO: 30), Ses iPR10-5 (SEQ ID NO: 10) and Ses iPR10-6 (SEQ ID NO: 29) was *de novo* generated by commercial gene synthesis, amplified by polymerase chain reaction (PCR) and subcloned into the expression pET24d, yielding vectors represented by SEQ ID NO12 (Ses iPR10-2), SEQ ID NO13 (Ses iPR10-3), SEQ ID NO14 (Ses iPR10-4), SEQ ID NO15 (Ses iPR10-5) and SEQ ID NO16 (Ses iPR10-6).

After transformation into *E. coli* and selection of positive clones as described in EP3754337, the Ses iPR-10 polypeptides, were expressed as His-tagged N-terminal fusion proteins represented by SEQ ID NO7 (Ses iPR10-2), SEQ ID NO8 (Ses iPR10-3), SEQ ID NO9 (Ses iPR10-4), SEQ ID NO10 (Ses iPR10-5) and SEQ ID NO11 (Ses iPR10-6) as described and purified by metal affinity chromatography (HiTrap IMAC HP) (product # 17092005, Cytvia). The purity was controlled by mass spectrometry and one dimensional sodium dodecyl sulfate polyacrylamide gel electrophoresis (1D-SDS-PAGE) (NP0321PK2, Thermofisher Scientific Inc.) 3 µg of the recombinant protein was analyzed as depicted in **Fig. 1****.**

### Example 2: Line blot assays

Unless otherwise stated, all described process steps are carried out at room temperature, 18 to 25 °C.

Line blots were manufactured as follows: Each of the inventive Ses iPR10 proteins were transferred as homogeneous lines to a membrane (nitrocellulose or nylon, (commercially available from Whatman)) by usage of a precision dispenser (commercially available from Zeta Corporation). According to manufactures instructions the blots were blocked, washed and cut into strips of 3 mm. An exemplary line blot is shown in **Fig. 2****.**

The EUROLINE strips were incubated as recommended in the manufacturer's (EUROIMMUN Medizinische Labordiagnostika AG, Lübeck, Germany) instructions (Product number DP 3110-1601 E). In Detail, the strips were incubated with 0,88 or 1,1 ml 1/11 diluted patient sample in working strength universal buffer (ZD 1129-0101 E, EUROIMMUN) for 16 hours. Subsequently the supernatants were aspirated off, the stripes washed three times for 5 min with 1 ml working strength universal buffer and then incubated for 1 h with anti-human IgE antibody conjugated to Alkaline Phosphatase (ZD 1129-0101 E, EUROIMMUN). Afterwards, the supernatants were aspirated off and the stripes were washed three times for 5 min with 1 ml working strength universal buffer again. For staining the strips were incubated for 10 min with 1 ml chromogen 5-bromo-4-chloro-3-indolyl phosphate (BCIP)/4-Nitro blue tetrazolium chloride (NBT) solution (ZD 1129-0101 E, EUROIMMUN) and after aspiring of the supernatant, the reaction was quenched with distilled water.

Blank controls were incubated in the same way, but working strength universal buffer only was incubated instead of diluted serum.

The strips were scanned and the signal intensities of the bands evaluated using the EUROLINEScan^{®} software (EUROIMMUN).

Pre characterized sera (n = 15) with IgE reactivity to sesame allergen extracts and of non sensitized patients (n = 10) without IgE reactivity to sesame allergen extracts were examined.

The results are summarized in Table 1, more specifically the number of samples reactive with sesame extract in which the presence of an antibody to the respective Ses iPR-10 allergen (*i.e*. Ses iPR-10-2, Ses iPR-10-3, Ses iPR-10-4, Ses iPR-10-5, Ses iPR-10-6) could be detected. In addition, the number of samples **not** reactive with sesame extract in which the presence of an antibody to the respective Ses iPR-10 allergen is shown.

| | Ses iPR10-2 | Ses iPR10-3 | Ses iPR10-4 | Ses iPR10-5 | Ses iPR10-6 |
|---|---|---|---|---|---|
| Number of positive samples reactive with sesame extract | 13(15) | 12(15) | 6(15) | 12(15) | 12(15) |
| Number of positive samples not reactive with sesame extract | 0/10 | 0/10 | 0/10 | 0/10 | 0/10 |

These results show that the inventors have now identified Ses iPR-10 allergens from sesame characterized by SEQ ID NO1 as an immunoreactive component of sesame extracts in patients. Five exemplary Ses iPR-10 allergens, referred to as Ses iPR-10-2, Ses iPR-10-3, Ses iPR-10-4, Ses iPR-10-5 and Ses iPR-10-6 were expressed and shown to be reactive using line assays.

Among the five Ses iPR-10 allergens, particularly strong reaction were detected when Ses iPR-2 and Ses iPR-6 were used.

Interestingly, at least two of the samples in the first group were reactive with at least one Ses iPR-10 allergen, but not with conventional allergens Bet v 1 and Cor a 1, indicating the teachings of the invention could also be used to increase the sensitivity of a method for aiding in the diagnosis of a PR-10 allergy.

### Example 3: Western blot assays

Unless otherwise stated, all described process steps are carried out at room temperature. For the Western Blot (WB) analysis 8 µg/cm of the recombinant Ses iPR10-2 or Ses iPR10-6 proteins (reduced) were separated in 4-12% gradient Bis-Tris-gels by 1D-SDS-PAGE (NP0321PK2, Thermofisher Scientific Inc.) under reducing conditions as described by the manufacturer and were blotted onto nitrocellulose membranes using the Fast Blotter system according to manufacturer (Thermo Fisher). The proteins were subsequently stained with Ponceau-S. (Ponceau S Solution (ab270042, abcam, Berlin, Germany). The membranes were cut into strips of 3 mm and washed 15 min with 0,4 ml working strength universal buffer. Sera were diluted 1/11 (v/v) with working strength universal buffer. Each strip was contacted separately with 0,4 ml of diluted patient sample for 16 h. The supernatants were aspirated off and the stripes were washed three times for 5 min with 0,4 ml working strength universal buffer. Subsequently, the strips were incubated with 0,4 ml anti-human IgE antibody conjugated to Alkaline Phosphatase (ZD 1129-0101 E, EUROIMMUN). Afterward, the supernatants were aspirated off and the stripes were washed again as described before. For staining strips were incubated 10 min with 0,4 ml chromogen 5-bromo-4-chloro-3-indolyl phosphate (BCIP)/4-Nitro blue tetrazolium chloride solution (ZD 1129-0101 E, EUROIMMUN). The reaction was quenched by incubation the strips with distilled water. Blank controls were incubated in the same way, but working strength universal buffer only was incubated instead of diluted serum.

For the His-Tag control strips were incubated 15 min with working strength universal buffer and after aspiring off with 0,4 ml anti-poly histidine antibody conjugated to Alkaline Phosphatase (ab81652, abcam) diluted 1:8000 (v/v) in working strength universal buffer. Afterward, the supernatants were aspirated off and the stripes were washed again as described before. For staining strips were incubated 10 min with 0,4 ml chromogen 5-bromo-4-chloro-3-indolyl phosphate (BCIP)/4-Nitro blue tetrazolium chloride solution (ZD 1129-0101 E, EUROIMMUN). The reaction was quenched by incubation the strips with distilled water.

The Strips were scanned and evaluated. The results are shown in **Fig 3****.**

Western Blot membrane stripes were incubated with patient samples (n = 8 for Ses iPR10-2 and n = 8 for Ses iPR10-6) of the reactive sera from Example 2. In samples from 7 out of 8 positive sera an antibody to Ses iPR10-2 could be detected. In samples from 8 out of 8 positive sera an antibody to Ses iPR10-6 could be detected.

Additionally, sera of sesame non-sensitized patients (n = 5) were incubated with the Western Blot membrane stripes. An antibody to at least one of Ses iPR10-2 and Ses iPR10-6 could be detected in none of the samples.

Separate Blank stripes were incubated without primary antibody and only contacted with the secondary conjugate antibody. An antibody to at least one of Ses iPR10-2 and Ses iPR10-6 could be detected in none of the samples.

Separate stripes for the His-Tag detection were incubated only with an anti-polyHistidine antibody. The polyHistidin-tag of Ses iPR10-2 and Ses iPR10-6 could be detected.

### Example 4: Line blot assays based on a larger cohort of patients

The experimental procedure outlined in example 2 was repeated using 30 characterized sera from patients sensitized against sesame. The results are shown in **Fig. 4****.** Negative sera were also used, but there was no reaction.

60 % of the patients' sera reacted with Ses iPR10-6, 33 % of the sera reacted with Ses iPR10-5, and 46 % of the sera reacted with Ses iPR10-2. Only weak reactions were observed with Ses iPR10-4 and Ses iPR10-3 (reactivity according to EAST class of no more than class 1).

All sera which showed a reaction to any of Ses iPR10-6, Ses iPR10-5 or Ses iPR10-2 were reactive with Ses iPR10-6, although some sera were more reactive with Ses iPR10-2 than with Ses iPR10-6. Only one patient showed reactivity according to EAST class 3 with Ses iPR10-3.

These results show that Ses iPR10-6 is the most reactive of the allergens used, followed by Ses iPR10-5 and Ses iPR10-2. Ses iPR10-6 may be combined with Ses iPR10-2 for an increased sensitivity compared to the use of Ses iPR10-6 alone.

### Example 5: Western blot analysis of Ses iPR variants

In order to demonstrate the reactivity of a variant derived from wild type sequences of Ses iPR10-2 and Ses iPR10-5, SEQ ID NO19 and SEQ ID NO20 respectively, example 3 was repeated using 14 sera from sesame-sensitized patients and five negative sera in addition to the wild type proteins of Ses iPR10-2, Ses iPR10-5 and Ses iPR10-6. Proteins were expressed as described in example 1.

The results are shown in **Fig. 5**. Reactivity could be demonstrated not only with the wild type sequences but also with the variants of Ses iPR10-2 and Ses iPR10-5. Preliminary data also showed a certain degree of reactivity with a Ses iPR10-6 variant (SEQ ID NO21).

The results demonstrate that not only wild type proteins but also variants may be used to practice the invention.

## Claims

1. A polypeptide comprising a Ses iPR-10 allergen from sesame **characterized by** SEQ ID NO1 or a variant thereof, wherein the polypeptide is isolated, purified or immobilized.

2. A diagnostically useful carrier comprising the polypeptide according to claim 1.

3. The carrier according to claim 1, wherein the carrier is selected from the group comprising a bead, a test strip, a microtiter plate, a plastic surface, preferably polystyrene surface, a branched cellulose-based polymer, a microarray, a blot, preferably from the group comprising western blot, line blot and dot blot, a glass surface, a slide, a biochip and a membrane, and is most preferably a microtiter plate or a line blot.

4. The polypeptide or carrier according to any of claims 2 to 3, wherein the polypeptide is in complex with an antibody from the sample of a patient, preferably an IgG, more preferably IgG4, or an IgE class antibody.

5. A kit comprising the carrier according to any of claims 2 to 4, wherein the kit further comprises one or more, preferably all from the group comprising a suitable water-tight vessel for contacting the diagnostically useful carrier with the sample, a negative control, positive control, a calibrator, preferably a set of calibrators, a washing buffer, a dilution buffer, a means for detecting an antibody to a Ses iPR-10 allergen from sesame **characterized by** SEQ ID NO1, preferably an IgG and/or IgE class antibody, wherein the antibody is preferably a mammalian, more preferably a human antibody, an antibody to a Ses iPR-10 allergen from sesame **characterized by** SEQ ID NO1, a means for capturing the antibody, preferably IgG and/or IgEs antibody, more preferably human, and a polypeptide comprising a Ses iPR-10 allergen from sesame **characterized by** SEQ ID NO1, preferably with a detectable label

6. The carrier or kit according to any of claims 1 to 5, wherein the carrier is a test stripe comprising spatially separate reagents which include a Ses iPR-10 allergen from sesame **characterized by** SEQ ID NO1 or a variant thereof, and one or more from the group comprising one or more calibrators, an anti-CCD-IgE control, a reagent indicating the presence of a secondary antibody, preferably a secondary antibody recognizing IgG, more preferably IgG4, or an IgE class antibodies, an indicator band and one or more calibration or cut off bands.

7. A pharmaceutical composition comprising a Ses iPR-10 allergen from sesame **characterized by** SEQ ID NO1 or a variant thereof, preferably further comprising an adjuvant.

8. A method for diagnosing or aiding in the diagnosis of an allergy comprising the step detecting in a sample from a subject the presence or absence of an antibody binding specifically to a Ses iPR-10 allergen from sesame **characterized by** SEQ ID NO1.

9. The method, wherein the sample is selected from the group comprising whole blood, saliva, serum and plasma.

10. A method comprising the step contacting a medical or diagnostic device comprising a polypeptide comprising a Ses iPR-10 allergen from sesame **characterized by** SEQ ID NO1 or a variant thereof with a buffered solution comprising an antibody binding specifically to a Ses iPR-10 allergen from sesame **characterized by** SEQ ID NO1, which solution is not a sample from a patient in need of a diagnosis comprising an unknown concentration of said antibody, wherein preferably
a) the concentration of the antibody in the solution is known, and/or
b) the antibody is a recombinant antibody and/or
c) the medical or diagnostic device is contacted with two or more solutions comprising the antibody, wherein the two or more solutions have a different concentration of the antibody, and/or
d) the antibody is recognized by secondary antibodies binding specifically to IgE class antibodies,
followed by detection of a signal which indicates whether or not the antibody has bound to the polypeptide, optionally a signal relating to the concentration of the antibody in the solution or solutions.

11. A use of a polypeptide comprising a Ses iPR-10 allergen from sesame **characterized by** SEQ ID NO1 or a variant thereof, the polypeptide, carrier or kit according to any of claims 1 to 6 for detecting an antibody binding specifically to a Ses iPR-10 allergen from sesame **characterized by** SEQ ID NO1.

12. A use of a polypeptide comprising a Ses iPR-10 allergen from sesame **characterized by** SEQ ID NO1 or an antibody binding specifically to a Ses iPR-10 allergen from sesame **characterized by** SEQ ID NO1 or the carrier according to any of claims 3, 4 or 6 for the manufacture of a diagnostic kit.

13. A use of a polypeptide comprising a Ses iPR-10 allergen from sesame **characterized by** SEQ ID NO1 or an antibody binding specifically to a Ses iPR-10 allergen from sesame **characterized by** SEQ ID NO1 or the carrier according to any of claims 3, 4 or 6 for diagnosing or aiding in the diagnosis of an allergy.

14. The carrier according to claim 4, the kit according to any of claims 5 or 6 or the method according to any of claims 8 to 10, wherein the antibody is an IgE or IgG class antibody.

15. The method or use according to any of claims 8 to 13, wherein the antibody is detected using a method from the group comprising immunodiffusion, immunoelectrophoresis, light scattering, agglutination, labeled immunoassays, preferably from the group comprising radiolabeled immunoassays, enzyme immunoassays such as colorimetric assays, chemiluminescence, preferably electrochemiluminescence immunoassays and immunofluorescence immunoassays.

16. An enriched, isolated, diluted or purified antibody, preferably IgE or IgG class antibody, or recombinant antibody binding specifically to a polypeptide a Ses iPR-10 allergen from sesame **characterized by** SEQ ID NO1, wherein the antibody is preferably in a solution comprising an artificial buffer or a natural buffer at a non-physiological concentration.
